(19) Europäisches Patentamt
European Patent Office
Office européen des brevets

(11) **EP 4 478 895 B1**

(12) **EUROPEAN PATENT SPECIFICATION**

(45) Date of publication and mention
of the grant of the patent:
**03.12.2025   Bulletin 2025/49**

(21) Application number: **23713303.8**

(22) Date of filing: **17.02.2023**

(51) International Patent Classification (IPC):
*A23K 20/105* (2016.01)     *A23K 20/20* (2016.01)
*A23K 20/24* (2016.01)     *A23K 50/15* (2016.01)
*A23K 20/111* (2016.01)     *A23K 20/137* (2016.01)
*A23K 20/22* (2016.01)     *A23K 50/10* (2016.01)

(52) Cooperative Patent Classification (CPC):
**A23K 50/15; A23K 20/105; A23K 20/111;
A23K 20/137; A23K 20/22; A23K 20/24;
A23K 50/10;** Y02P 60/22

(86) International application number:
**PCT/EP2023/054080**

(87) International publication number:
**WO 2023/156618 (24.08.2023 Gazette 2023/34)**

(54) **A MIXTURE OF UREA, BIURET AND N-CONTAINING BY-PRODUCTS CREATED DURING BIURET PRODUCTION AS A DIETARY NON-PROTEIN NITROGEN-SOURCE FOR RUMINANTS AND USES THEREOF**

MISCHUNG AUS HARNSTOFF, BIURET UND N-HALTIGEN NEBENPRODUKTEN BEI DER BIURETHERSTELLUNG ALS DIÄTETISCHE NICHTPROTEINISCHE STICKSTOFFQUELLE FÜR WIEDERKÄUER UND VERWENDUNGEN DAVON

MÉLANGE D'URÉE, DE BIURET ET DE SOUS-PRODUITS CONTENANT DU N CRÉÉS PENDANT LA PRODUCTION DE BIURET EN TANT QUE SOURCE D'AZOTE NON PROTÉIQUE ALIMENTAIRE POUR RUMINANTS ET SES UTILISATIONS

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **17.02.2022   EP 22157277
03.06.2022   EP 22382539
09.09.2022   EP 22382836**

(43) Date of publication of application:
**25.12.2024   Bulletin 2024/52**

(73) Proprietor: **Yara International ASA
0277 Oslo (NO)**

(72) Inventors:
• **RUIZ, Isabel
28806 Alcalá de Henares, Madrid (ES)**
• **MARTINEZ-LUENGAS, Inés
28223 Pozuelo de Alarcón, Madrid (ES)**
• **PIRRO, Laura
4541 HJ Terneuzen (NL)**
• **VAN BELZEN, Ruud
4541 HJ Terneuzen (NL)**
• **MOHAN, Anand
4541 HJ Terneuzen (NL)**
• **IPHARRAGUERRE, Ignacio R.
08461 Sant Esteve de Palautordera, Barcelona
(ES)**

(74) Representative: **De Clercq & Partners
Edgard Gevaertdreef 10a
9830 Sint-Martens-Latem (BE)**

(56) References cited:
**CA-A- 1 241 663     US-A- 2 768 895**

**(Cont. next page)**

- XIUMIN ZHANG ET AL: "Effects of urea plus nitrate pretreated rice straw and corn oil supplementation on fiber digestibility, nitrogen balance, rumen fermentation, microbiota and methane emissions in goats", JOURNAL OF ANIMAL SCIENCE AND BIOTECHNOLOGY, BIOMED CENTRAL LTD, LONDON, UK, vol. 10, no. 1, 23 January 2019 (2019-01-23), pages 1 - 10, XP021270875, DOI: 10.1186/S40104-019-0312-2

**Description**

**Technical field**

The present disclosure relates to the field of dietary non-protein nitrogen (NPN)-sources, more in particular a mixture of urea, biuret and N-containing by-products created during the urea condensation process, or stated differently, during biuret production, in a ruminant feed supplement.

**Background**

Ruminants are defined as large hoofed herbivorous grazing or browsing mammals that are able to acquire nutrients from plant-based feed by digesting it in a specialized stomach having four compartments, one of which is the rumen. Thanks to the microbial actions in the rumen prior to digestion, ruminants have the unique ability to use (low quality) protein sources and also non-protein nitrogen (NPN) sources. NPN sources provide nitrogen for ruminants, especially in low-protein diets. This gives ruminant animals a competitive advantage over other domesticated animals. The symbiotic relationship with the rumen microflora allows ruminants to upcycle nutrients to produce highly nutritious animal proteins, by means of an efficient pre-gastric fermentation of feeds, which takes place in the reticulorumen, the largest compartment of their gastrointestinal tract. Ruminants provide an optimum environment for microorganisms to thrive, and they, in turn, supply the ruminant host with microbial crude protein, which is of high quality in terms of amino acids supplement. In beef and dairy production systems, the microbial crude protein synthesized in the rumen provides the majority of the metabolizable protein required by the animal.

Increased utilization of nitrogen (N) for bacterial synthesis and capture of dietary N into anabolic products, particularly absorbed amino acids, may be influenced by dietary manipulation, mainly by energy levels in the ration. However, there is an upper limit to the overall efficiency of the process: a maximum of 50 to 60% of dietary N, or 70 to 90% of apparent digested N will be converted into amino acids and released into the portal vein. For this reason, particularly in beef cattle diets, it is extremely important to assess the optimum kinetics of N release in NPN sources for each energy source (e.g., basal forage diet, supplemental by-products, etc.) to maximize N utilization and minimize excretion.

In most beef production systems, several grains and grain byproducts are available as supplemental protein sources. However, on a per kg of N supplied, it is nearly impossible to compete with the lower costs of supplemental crude protein in the form of NPN.

Indeed, non-protein nitrogen, mainly in the form of urea, is known as a cost-effective source of supplemental nitrogen (N) in ruminant production. Commercial/feed-grade urea typically contains only traces of biuret, with a biuret content not exceeding 1.0 wt%. Urea is however usually not included in more than 1 wt.% of the diet. Any shortage of protein in a diet or supplementation program is frequently covered by the use of urea-based supplements in the form of range cubes, liquid or pelleted supplements and tubs. Apart from urea, other ammonia forming chemical compounds such as ammonium sulfate have been used in the formulation of ruminal diets to replace part of plant protein sources and providing adequate amounts of ruminal degradable protein for a better efficiency of fiber digestion and the synthesis of microbial protein. The use of NPN, however, has been traditionally limited by the fast rate of N-release in the rumen of a ruminant (animal) in the form of ammonia. If the release of ammonia in the rumen exceeds the use capacity by ruminal microbiota, this N-excess will be excreted, with a consequent loss of energy and N-efficiency. Furthermore, if the ammonia concentration overpasses the liver excretion capacity of the ruminant, ammonia toxicity or death may occur.

As a result, in the prior art, it has been aimed at improving the use of NPN to reduce the rate of N-release in the rumen. In recent years, several slow-release urea products have been developed and tested in ruminant nutrition, for instance by physical means, such as by applying a coating, with variable results. Applying a coating with the aim to obtain slow release means adding fat, wax or the like to the NPN-source. This has the disadvantage that the NPN-content is diluted. A coating furthermore increases the production cost of a dietary N-source in cattle diets that should be cost effective. Another solution is to apply chemical means such as using pure biuret. The disadvantage of pure biuret is that the production thereof is very expensive, because the heating time to obtain the pure biuret out of urea has to be extended a lot.

In addition, currently, there is growing interest in decreasing the environmental impact of ruminant production, including beef and dairy, and many strategies are being pursued to achieve this goal.

In the current ruminant feeding systems, methane ($CH_4$) production is an inevitable consequence of fermentative digestion. The microbial consortium of the rumen must dispose hydrogen that is produced during fermentation. Otherwise, hydrogen would accumulate in the rumen and thereby inhibit fermentation of ingested feeds. Therefore, the rumen microbial system is provided with methanogens that reduce carbon dioxide ($CO_2$) to methane.

The major hydrogen (electron) sink in the rumen is methane produced by the reduction of carbon dioxide using reduced co-enzymes such as NADH as the electron source. Methane is however an energy loss for ruminants and a potent greenhouse gas, which has a 28-times greater global warming potential than carbon dioxide.

Globally, ruminants produce some 80 million tons of methane annually, accounting for some 28% of manmade methane emission. The adoption of ruminant management practices which result in major reductions of release of methane are consequently high priority.

In the article with the title "Effects of urea plus nitrate pretreated rice straw and corn oil supplementation on fiber digestibility, nitrogen balance, rumen fermentation, microbiota and methane emissions in goats", Xiumin Zhang et al., Journal of Animal Science and Biotechnology, Biomed, Central Ltd., London, UK, vol. 10, no. 1, 23 January 2019, page 1 - 10, a study is described where the combined effects of urea plus nitrate pretreated rice straw and corn oil supplementation to the diet on nutrient digestibility, nitrogen (N) balance, CH4 emissions, ruminal fermentation characteristics and microbiota in goats are examined.

It is a goal of the present disclosure to provide NPN-sources for ruminants, such as for a ruminant feed supplement composition which overcomes the limitations of the known prior art, useful for improving the rumen fermentation, the productivity and the performance of the ruminant animals, and the enteric methane emission, especially in low crude protein diets, which provides N in a timely manner such that the microbial growth in the rumen is enhanced and the production of energy yielding compounds is improved.

**Summary**

The invention is defined by the appended claims.

The present disclosure generally relates to a composition or mixture comprising biuret, urea, and N-containing by-products of the biuret production out of urea, and uses thereof and methods related thereto. The terms "NPN composition", "NPN mixture", and "composition comprising urea, biuret and N-containing by-products of the biuret production out of urea" are generally used interchangeably herein, and refer to a composition or NPN composition according to the present disclosure, as further detailed herein.

According to a first aspect of the present disclosure, the use of a mixture or composition of biuret, urea and N-containing by-products of the biuret production out of urea as a NPN-source in a ruminant feed supplement composition to reduce the methane emission produced by a ruminant animal is disclosed. It has been observed that the supplementation of a ruminant feed with urea, biuret and N-containing by-products of the biuret production out of urea as a NPN source, reduces enteric methane production and emission of ruminant animals without compromising or even improving their performance, in particular when compared to a diet or ruminant feed supplemented with urea as NPN source.

According to a second aspect of the present disclosure, the use of a mixture of biuret, urea and minor N-containing compounds created during the urea condensation process as a NPN-source in a ruminant feed supplement composition to decrease the acetate to propionate ratio produced by rumen microbes in a ruminant has been disclosed. Stated differently, the use of a composition comprising biuret, urea and N-containing by-products of the biuret production out of urea as a NPN-source in a ruminant feed supplement composition to decrease the acetate to propionate ratio produced by rumen microbes in a ruminant is disclosed, in particular when compared to a diet or ruminant feed supplemented with urea as NPN source.

According to a third aspect of the present disclosure, the use of a mixture of biuret, urea and minor N-containing compounds created during the urea condensation process as a NPN-source in a ruminant feed supplement composition to improve the efficiency of microbial protein synthesis (EMPS) has been disclosed. Stated differently, the use of a composition comprising biuret, urea and N-containing by-product of the biuret production out of urea as a NPN-source in a ruminant feed supplement composition to improve the efficiency of microbial protein synthesis (EMPS) is disclosed, in particular when compared to a diet or ruminant feed supplemented with urea as NPN source.

According to a fourth aspect of the present disclosure, the use of a mixture of biuret, urea and minor N-containing compounds created during the urea condensation process as a NPN-source in a ruminant feed supplement composition to increase the dry matter (DM) and organic matter (OM) digestibility has been disclosed. Stated differently, the use of a composition comprising biuret, urea and N-containing by-products of the biuret production out of urea as a NPN-source in a ruminant feed supplement composition to increase the dry matter (DM) and organic matter (OM) digestibility is disclosed, in particular when compared to a diet or ruminant feed supplemented with urea as NPN source.

According to a further aspect of the present disclosure, the use of a ruminant feed supplement composition comprising urea, biuret and N-containing by-products of the biuret production out of urea as a NPN-source to improve the dry matter intake (DMI) of ruminant animals is disclosed, in particular when compared to a diet or ruminant feed supplemented with urea as NPN source.

It has now unexpectedly been discovered by the applicant that by using a combination of urea, biuret and minor N-containing compounds created during the urea condensation process to form the biuret (also referred to as "by-products of the biuret production based on urea" or "N-containing by-products of the biuret production out of urea"), the rumen fermentation is altered directly in a way that the enteric methane production is decreased, the acetate to propionate ratio is decreased, as well as that the efficiency of microbial protein synthesis is improved. It has furthermore been found that the DM and OM digestibility is increased, and that the dry matter intake of the ruminant animal is increased, in particular when compared to a diet or ruminant feed supplemented with urea as NPN source.

According to a possible use according to the present disclosure, the mixture or composition comprises between 30 - 60 wt.% of urea and between 30 and 60 wt.% biuret, with wt% based on the total weight of the mixture or composition. More in particular, the mixture or composition comprises between 35 and 55 wt.% of urea and between 35 and 50 wt.% of biuret, with wt% based on the total weight of the composition. Even more in particular, the mixture or composition comprises between 37 and 52 wt.% of urea and between 39 and 46 wt.% of biuret, with wt% based on the total weight of the composition.

In a particular use according to the present disclosure, the composition comprises between 5 and 30 wt.% of N-containing by-products of the biuret production out of urea, based on the total weight of the composition.

In a specific use according to the present disclosure, the ruminant feed supplement composition further comprises:

- between 2.5 and 8 wt.% of triuret, and/or
- between 0.1 and 5 wt.% of ammelide, and/or
- between 2.5 and 15 wt.% of cyanuric acid, and
- between 0.1 and 2.0 wt.% of moisture,

based on the total weight of the composition.

According to a further specific use according to the present disclosure, the mixture further comprises

- between 3 and 6 wt.% of triuret, and/or
- between 0.5 and 3 wt.% of ammelide, and/or
- between 3 and 10 wt.% of cyanuric acid, and
- between 0.1 and 1.5 wt.% of moisture.

Another aspect of the present disclosure provides a ruminant feed composition comprising one or more nutritional ingredients and an NPN comprising biuret, urea and N-containing by-products of the biuret production out of urea. In certain embodiments, the NPN composition comprises (a) between 30 and 60 wt.% of urea; (b) between 30 and 60 wt.% of biuret; and (c) between 5 wt.% and 30 wt.% of N-containing by-products of the biuret production out of urea, based on the total weight of the NPN composition. More in particular, the NPN composition comprises (a) between 37 and 52 wt.% of urea; (b) between 39 and 46 wt.% of biuret; (c) between 3 wt.% and 6 wt.% of triuret; (d) between 0.5 and 3 wt.% of ammelide; and (e) between 3 and 10 wt.% of cyanuric acid, based on the total weight of the NPN composition. In certain embodiments, the one or more nutritional ingredients form a nutritionally balanced ration or a basal ration for a ruminant.

According to a further related aspect of the present disclosure, a method for reducing the enteric methane emissions of a ruminant animal is disclosed, said method comprising administering to the ruminant animal a NPN composition according to the present disclosure, comprising biuret, urea and N-containing by-products of the biuret production out of urea, wherein the composition comprises between 30 and 60 wt.% of urea and between 30 and 60 wt.% biuret. It has been found that the enteric methane emissions of the ruminant animal, expressed as g methane/kg dry matter intake or g methane/kg organic matter intake, is reduced by at least 5%, particularly by at least 10%, such as by about 15%, when the NPN composition is administered to a ruminant as NPN source compared to a diet comprising urea as NPN source.

According to a further related aspect of the present disclosure, a method for decreasing the acetate-to-propionate ratio produced by rumen microbes in a ruminant animal is disclosed, said method comprising administering to the ruminant animal a NPN composition according to the present disclosure, comprising biuret, urea and N-containing by-products of the biuret production, wherein the NPN composition comprises between 30 and 60 wt.% of urea and between 30 and 60 wt.% biuret. It has been found that the acetate-to-propionate ratio produced by rumen microbes in a ruminant, particularly estimated via an *in vitro* fermentation, such as a continuous flow fermentation with rumen fluid to simulate rumen fermentation, is decreased by at least 5%, in particular by at least 10%, such as by about 20%, when the NPN composition is administered to a ruminant as NPN source compared to a diet comprising urea as NPN source.

According to a further related aspect of the present disclosure, a method for improving the efficiency of microbial protein synthesis (EMPS) by rumen microbes in a ruminant animal is disclosed, said method comprising administering to the ruminant animal a NPN composition according to the present disclosure comprising biuret, urea and N-containing by-products of the biuret production out of urea, wherein the NPN composition comprises between 30 and 60 wt.% of urea and between 30 and 60 wt.% biuret. It has been found that the EMPS, particularly estimated via an *in vitro* fermentation with rumen fluid to simulate rumen fermentation was increased by at least 5%, in particular by at least 10%, when the NPN composition is administered to a ruminant as NPN source compared to a diet comprising urea as NPN source.

According to a further related aspect of the present disclosure, a method for increasing the dry matter (DM) and organic matter (OM) digestibility is disclosed, said method comprising administering to the ruminant animal a NPN composition according to the present disclosure comprising biuret, urea and N-containing by-products of the biuret production, wherein the NPN composition comprises between 30 and 60 wt.% of urea and between 30 and 60 wt.% biuret.

According to a further related aspect of the present disclosure, a method for increasing the dry matter intake (DMI) of a ruminant animal, said method comprising administering to the ruminant animal a NPN composition according to the present disclosure comprising biuret, urea and N-containing by-products of the biuret production, wherein the NPN composition comprises between 30 and 60 wt.% of urea and between 30 and 60 wt.% biuret. It has been found that the DMI, expressed in kg DM/day, is increased by at least 5% when the (NPN) composition is administered to a ruminant as NPN source compared to a diet comprising urea as NPN source.

In particular embodiments, the NPN composition according to the present disclosure and administered in the methods according to the present disclosure comprises between 35 and 55 wt.% of urea and between 35 and 50 wt.% of biuret, based on the total weight of the composition, more in particular comprises between 37 and 52 wt.% of urea and between 39 and 46 wt.% of biuret, based on the total weight of the composition.

In particular embodiments, the NPN composition according to the present disclosure and administered in the methods according to the present disclosure further comprises between 5 and 30 wt.% of N-containing by-products of the biuret production out of urea, based on the total weight of the composition.

In particular embodiments, the NPN composition according to the present disclosure and administered in the methods according to the present disclosure comprises between 2.5 and 8 wt.% of triuret, and/or between 0.1 and 5 wt.% of ammelide, and/or between 2.5 and 15 wt.% of cyanuric acid, and between 0.1 and 2.0 wt.% of moisture, based on the total weight of the composition, more in particular, wherein the composition comprises between 3 and 6 wt.% of triuret, and/or between 0.5 and 3 wt.% of ammelide, and/or between 3 and 10 wt.% of cyanuric acid, and between 0.1 and 1.5 wt.% of moisture, based on the total weight of the composition.

In certain embodiments, the methods according to the present disclosure further comprises the steps of preparing or providing a ruminant feed supplement composition comprising the NPN composition, or preparing or providing a ruminant feed composition comprising the NPN composition, and administering the ruminant feed supplement or the ruminant feed composition to the ruminant animal. More in particular, the NPN composition, comprising biuret, urea and N-containing by-products of the biuret production according to the present disclosure is present in the ruminant feed composition in a concentration between 0.5 and 4 wt.%, more in particular between 0.5 and 3.5 wt.% or between 0.5 and 3 wt.%, even more in particular between 1 and 2.5 wt.%, with wt% based on the total dry matter basis of the ruminant feed composition. In particular, the ruminant feed composition further comprises one or more nutritional ingredients, wherein the one or more nutritional ingredients form a nutritionally balanced ration or a basal ration for a ruminant animal.

It is understood that the different methods according to the present disclosure are non-therapeutic methods.

### Description of the figures

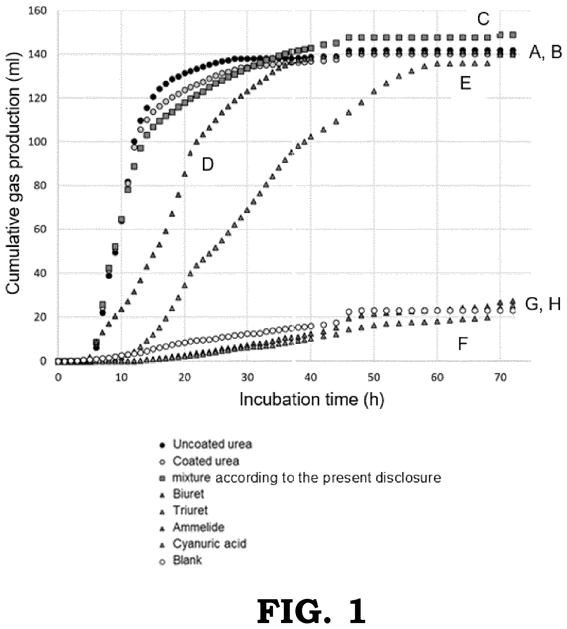

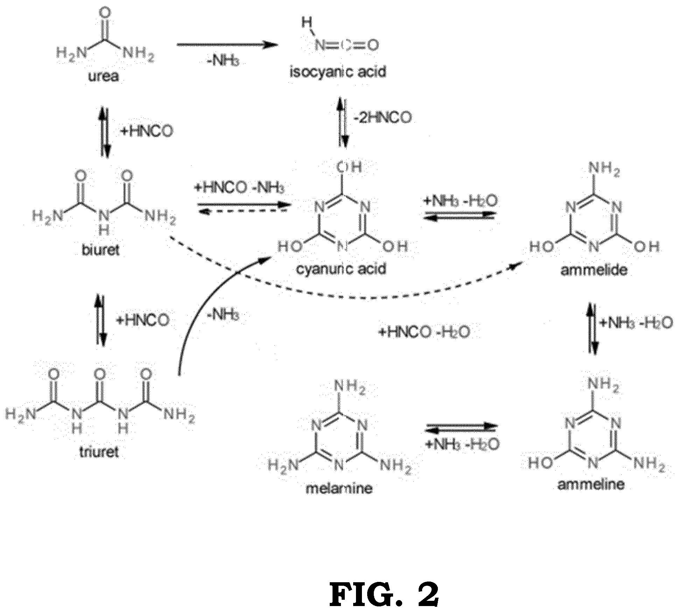

FIG. 1 shows a graph of the cumulative gas production of 6 different nitrogen-containing original products, in particular uncoated urea (A), coated urea (B), biuret (D), triuret (E), ammelide (F) and cyanuric acid (G), and a mixture according to the present disclosure (C) on an isonitrogenous base against a blank (H) during 72 hours of *in vitro* incubation. FIG. 2 shows an overview of the reactions involved in the production of biuret from urea, including the formation of N-containing by-products.

### Detailed description

The present inventors have surprisingly found that a specific NPN composition (or mixture) for ruminants, comprising urea, biuret and one or more N-containing by-products of a urea condensation process or biuret production process, has many advantages when fed to a ruminant as a NPN source, particularly when compared to a ruminant fed with urea as a NPN source. Moreover, for the different embodiments and aspects envisaged herein, the NPN composition or NPN mixture according to the present disclosure significantly outperforms urea as a NPN source (when compared to a ruminant fed with a diet without NPN source).

The advantages of a NPN composition according to the present disclosure are in particular:

(1) reduction of the enteric methane emissions by ruminant animals. The reduction of the methane production or emissions by ruminant animals can be assessed *in vivo,* by determining the methane emissions of a ruminant fed with a particular diet, for instance using using the $SF_6$ tracer technique as e.g. described in example 3, wherein the methane emissions may be expressed as g methane emitted per kg dry matter intake or per kg organic matter intake. Alternatively, the methane emissions may be assessed by evaluating the methane production in *in vitro* culture incubations of ruminal fluid, as e.g. described in example 4, wherein the methane production may be expressed as mM, ml or mg methane produced per g of organic matter incubated or digested;

(2) the acetate to propionate ratio produced by the rumen microbes was decreased. The acetate and propionate content can be determined by gas chromatography determination of the volatile fatty acids (VFA) in a sample, as known by the skilled person. In certain embodiments, the sample is obtained via an *in vitro* fermentation with ruminant fluid, to simulate rumen fermentation, particularly wherein a suitable fermenter or incubator is inoculated with ruminal fluid and fed with a ruminant feed composition to simulate rumen fermentation;

(3) the efficiency of microbial protein synthesis (EMPS) was increased. As understood by the skilled person, the EMPS is a measure for how much microbial protein is produced due to the microbial activity for every unit of fermentable substrate which is fed to the ruminant. It may be determined by an *in vitro* assay comprising continuous flow or batch fermentations with rumen fluid, particularly wherein a suitable fermenter or incubator is inoculated with ruminal fluid and fed with a ruminant feed composition to simulate rumen fermentation, as for instance explained in example 2 or 4;

(4) the dry matter (DM) and organic matter (OM) digestibility was increased. The DM and OM digestibility may be assessed by the difference in dry matter and organic matter (i.e. dry matter minus the ash content) of the feed vs the sample after (in vitro) fermentation, as known by the skilled person;

(5) the rumen fermentation was increased after 72h in an *in vitro* assay;

(6) the dry matter intake (DMI) of ruminant animals was increased.

In this context, and without wishing to be bound by theory, it is believed that there has been a selective pressure on the microbes in the rumen that produce acetate and propionate in such a way that more propionate and less acetate, in view of ruminant feed supplement compositions without the mixture according to the present disclosure, are produced. Propionate is the most important glucogenic precursor in ruminants. This means that lowering the acetate to propionate ratio enhances the supply of energy for metabolic use, through which more energy is available to be used by the ruminant enhancing its production of proteins. In other words, the rumen fermentation has been made more efficient in an energetic way, supporting the production of meat, milk, etc.

Biuret is typically formed through a non-catalytic thermal decomposition of urea. This process is generally referred to herein as "biuret production", "biuret production out of urea" or "urea condensation process". The main reaction and the side reactions in biuret production out of urea are known to the skilled person and they are represented in FIG. 2. When urea is heated to temperatures above its melting point, ammonia is slowly evolved. If the temperature range is limited slightly above the normal melting point of urea, i.e. 132 °C, biuret is formed. When heating the urea melt further, several other different "N-containing compounds" or "N-containing by-products" are formed in very small amounts - hence use of the term "minor". These N-containing compounds resulting out of the thermal decomposition of urea encompass amongst others cyanuric acid, triuret, ammelide, etc. These compounds are referred to herein as "N-containing by-products of biuret production" or as "minor N-containing compounds created during the urea condensation process". In particular embodiments, said N-containing by-products of biuret production comprise triuret, ammelide and cyanuric acid. These by-products are poorly hydrolysed in the rumen. Cyanuric acid and triuret are, just like biuret, non-toxic for the ruminants and give as good nitrogen retention figures as biuret does. Ammelide is an unavoidable by-product in the production of the necessary amount of biuret. The amount of ammelide however needs to be limited since it can be toxic in higher doses.

As used herein, the composition or mixture according to the present disclosure, in particular the NPN composition or mixture according to the present disclosure, generally comprises urea, biuret and one or more by-products of a biuret production process. More in particular, the NPN composition or mixture according to the present disclosure, comprises between 60 wt.% and 95 wt.% of urea and biuret, and between 5 wt.% and 40 wt.% of N-containing by-products of a biuret production process, based on the total weight of the composition or mixture. Even more in particular, the NPN composition or mixture according to the present disclosure comprises between 70 wt.% and 95 wt.% or between 75 wt.% and 95 wt.% of urea and biuret, and between 5 wt.% and 30 wt.% or between 5 wt.% and 25 wt.% of N-containing by-products of a biuret production process, based on the total weight of the composition or mixture.

In particular embodiments, the NPN composition or mixture according to the present disclosure comprises between 30 and 60 wt.% of urea and between 30 and 60 wt.% of biuret, more in particular between 35 and 55 wt.% of urea and between 35 wt.% and 50 wt.% of biuret, even more in particular between 37 and 52 wt.% or between 40 and 50 wt.% of urea and between 39 and 46 wt.% or between 40 and 45 wt.% of biuret, based on the total weight of the composition or mixture. In certain embodiments, the NPN composition or mixture according to the present disclosure, further comprises between 5 wt.% and 30 wt.% of N-containing by-products of a biuret production process out of urea, in particular between 5 wt.% and 25 wt.% of N-containing by-products of a biuret production process out of urea, based on the total weight of the composition or mixture. In certain embodiments the N-containing by-products of a biuret production process out of urea comprises one or more of triuret, ammelide and cyanuric acid.

In certain embodiments, the NPN composition or NPN mixture according to the present disclosure comprises, as N-containing by-products of a biuret production process, (a) between 2.5 wt.% and 8 wt.% of triuret, more in particular between 3 and 6 wt.% of triuret; (b) between 0.1 and 5 wt.% of ammelide, more in particular between 0.5 and 3 wt.% of ammelide; and/or (c) between 2.5 and 15 wt.% of cyanuric acid, more in particular between 3 and 10 wt.% of cyanuric acid, based on the total weight of the composition or mixture.

In certain embodiments, the NPN composition or mixture according to the present disclosure further comprises between 0.1 and 2 wt.% moisture (water), more in particular between 0.1 and 1.5 wt.% moisture, based on the total weight of the composition or mixture.

In particular embodiments, he NPN composition or mixture according to the present disclosure is a ruminant feed supplement composition.

Particular embodiments of the NPN composition or mixture or ruminant feed supplement composition according to the present disclosure comprises

- between 30 and 60 wt.% of urea, more in particular between 35 and 55 wt.% of urea, even more in particular between 37 and 52 wt.% of urea,
- between 30 and 60 wt.% of biuret, more in particular between 35 and 50 wt.% of biuret, even more in particular between 39 and 46 wt.% of biuret,
- between 3 and 6 wt.% of triuret,
- between 0.5 and 3 wt.% of ammelide,
- between 3 and 10 wt.% of cyanuric acid,
- between 0.1 and 1.5 wt.% of moisture, with wt.% based on the total weight of the composition.

Optionally, for all embodiments of the NPN composition according to the present disclosure, one or more anti-caking agents as generally known in the art can be added to the ruminant feed supplement composition, either incorporated in the feed supplement, either as a coating, to improve handling properties and to improve the quality of the ruminant feed supplement composition.

Furthermore, it is also possible to add any pre-biotics, pro-biotics, post-biotics, antibiotics, bio stimulants known in the art for fostering/supporting the nitrogen assimilation and rumen fermentation.

An aspect of the present disclosure relates to the use of a NPN composition or mixture according to the present disclosure, or stated differently a mixture or composition of urea, biuret and minor N-containing compounds created during the urea condensation process to obtain the biuret, as a NPN-source in a ruminant feed supplement composition to, particularly at the same time or simultaneously,

- decrease the enteric methane emissions of a ruminant animal,
- improve the dry matter intake of a ruminant animal,
- decrease the acetate to propionate ratio produced by rumen microbes in a ruminant,
- improve the EMPS (efficiency of microbial protein synthesis), and
- increase the DM and OM digestibility.

According to a related aspect of the present disclosure, a composition comprising urea, biuret and N-containing by-products of biuret production out of urea, for use as an NPN-source in a ruminant feed supplement composition to decrease the enteric methane production or emissions of a ruminant animal or to decrease the enteric methane production by rumen microbes in a ruminant is disclosed.

According to a related aspect of the present disclosure, a composition comprising urea, biuret and N-containing by-products of biuret production out of urea, for use as an NPN-source in a ruminant feed supplement composition to decrease the ratio of acetate to propionate produced by rumen microbes in a ruminant is disclosed.

According to a further related aspect of the present disclosure, a composition comprising urea, biuret and N-containing by-products of biuret production out of urea, for use as an NPN-source in a ruminant feed supplement composition to improve the EMPS in a ruminant is disclosed.

According to a further related aspect of the present disclosure, a composition comprising urea, biuret and N-containing by-products of biuret production out of urea, for use as an NPN-source in a ruminant feed supplement composition to increase the DM and OM digestibility in a ruminant.

According to a related aspect of the present disclosure, a composition comprising urea, biuret and N-containing by-products of biuret production out of urea, for use as an NPN-source in a ruminant feed supplement composition to increase the dry matter intake of a ruminant animal is disclosed.

A related aspect of the present disclosure provides a method for reducing the enteric methane production of a ruminant animal, comprising the steps of

- preparing a ruminant feed composition comprising a NPN composition according to the present disclosure, as a NPN source, particularly a NPN composition comprising biuret, urea and N-containing by-products of the biuret production as disclosed herein; and
- feeding a ruminant with the ruminant feed composition.

A related aspect of the present disclosure provides a method to decrease the acetate to propionate ratio produced by rumen microbes in a ruminant, comprising the steps of:

- preparing a ruminant feed composition comprising a NPN composition according to the present disclosure, as a NPN source, particularly a NPN composition comprising biuret, urea and N-containing by-products of the biuret production as disclosed herein; and
- feeding a ruminant with the ruminant feed composition.

A further related aspect of the present disclosure provides a method to improve the EMPS in a ruminant, comprising the steps of

- preparing a ruminant feed composition comprising a NPN composition according to the present disclosure, as a NPN source, particularly a NPN composition comprising biuret, urea and N-containing by-products of the biuret production as disclosed herein; and
- feeding a ruminant with the ruminant feed composition.

A further related aspect of the present disclosure relates to a method to increase the DM and OM digestibility in a ruminant, comprising the steps of

- preparing a ruminant feed composition comprising a NPN composition according to the present disclosure, as a NPN source, particularly a NPN composition comprising biuret, urea and N-containing by-products of the biuret production as disclosed herein; and
- feeding a ruminant with the ruminant feed composition.

In certain embodiments, the enteric methane emissions of the ruminant animal are reduced by at least 5% or by at least 10%, such as by about 15%, when expressed in g/kg DMI (dry matter intake) or g/kg OMI (organic matter intake), when the NPN composition according to the present disclosure is administered to a ruminant as NPN source compared to diet comprising urea as NPN source.

In certain embodiments the acetate to propionate ratio produced by rumen microbes in a ruminant is decreased by at least 5%, in particular by at least 10%, such as by about 20%, when the NPN composition according to the present disclosure is administered to a ruminant as NPN source compared to a diet comprising urea as NPN source. In particular, the acetate-to-propionate ratio may be determined on a sample obtained from an *in vitro* fermentation with ruminant fluid, to simulate rumen fermentation, particularly wherein a suitable fermenter or incubator, such as a continuous flow or batch fermenter or incubator, is inoculated with ruminal fluid and fed with a ruminant feed composition to simulate rumen fermentation.

In certain embodiments, the efficiency of microbial protein synthesis (EMPS) in a ruminant is increased by at least 5%, in particular by at least 10%, such as by about 20%, when the NPN composition according to the present disclosure is administered to a ruminant as NPN source compared to a diet comprising urea as NPN source. In particular, the EMPS may be determined on a sample obtained from an *in vitro* fermentation with ruminant fluid, to simulate rumen fermentation, particularly wherein a suitable fermenter or incubator, such as a continuous flow or batch fermenter or incubator, is inoculated with ruminal fluid and fed with a ruminant feed composition to simulate rumen fermentation.

In certain embodiments, the method for decreasing the enteric methane production of a ruminant animal, for decreasing the acetate to propionate ratio produced by rumen microbes in a ruminant, for improving the EMPS in a ruminant, for increasing the DM or OM digestibility in a ruminant, and/or for increasing the dry matter intake (DMI) of a ruminant further comprises the step of replacing a portion of vegetable proteins in the feed composition with the NPN composition according to the present disclosure.

It is further understood that the different methods according to the present disclosure are non-therapeutic methods.

As considered herein a ruminant or ruminant animal is a bovine, ovine or caprine animal, particularly a domesticated animal with a functional rumen. In particular embodiments, the ruminant is a sheep or a cattle, more in particular the ruminant is a beef cattle or dairy cattle.

The present disclosure further provides a feed, feed material, premix or feed additive for feeding a ruminant comprising the NPN composition according to the present disclosure. The NPN composition according to the present disclosure may be combined with a ruminant feed or feed material, or with a premix for feeding a ruminant. In context of the present disclosure the term premix or nutrient premix is used as known to the person skilled in the art and denotes a mixture comprising one or more ingredients such as vitamins, trace minerals, medicaments, feed supplements and diluents. A ruminant feed or ruminant feed material generally refers to nutritional compositions suitable for feeding to ruminants, as known to the skilled person, and includes compound feeds (i.e. generally an industrially produced feed mixture of at least two feed materials), complete feeds (i.e. a compound feed containing all main nutrients needed for a daily ration, including also all the roughage needed by the ruminant), or a concentrate feed (i.e. a compound feed which has a high content of certain substances but which is sufficient for a daily ration only if used in combination with other feed).

In particular embodiments, a ruminant feed composition comprising one or more nutritional ingredients and a NPN composition comprising (a) between 30 and 60 wt.% or between 35 and 55 wt.% of urea; (b) between 30 and 60 wt.% or between 35 and 50 wt.% of biuret; and (c) between 5 wt.% and 30 wt.% of N-containing by-products of the biuret production out of urea, based on the total weight of the NPN composition, is disclosed. More in particular, the NPN composition comprises (a) between 37 and 52 wt.% of urea; (b) between 39 and 46 wt.% of biuret; (c) between 3 wt.% and 6 wt.% of triuret; (d) between 0.5 and 3 wt.% of ammelide; and (e) between 3 and 10 wt.% of cyanuric acid, based on the total weight of the NPN composition. In certain embodiments, the one or more nutritional ingredients form a nutritionally balanced ration or a basal ration for a ruminant, as known by the skilled person.

In certain embodiments, a ruminant feed composition or feed material comprising between 0.5 and 4 wt.%, more in particular between 0.5 and 3.5 wt.% or between 0.5 and 3 wt.%, even more in particular between 1 and 2.5 wt.% of t NPN composition according to the present disclosure, is disclosed, with wt.% based on the total dry matter of the feed composition or feed material,

The present disclosure further relates to a method for feeding or rearing a ruminant comprising administering the NPN composition according to the present disclosure to the ruminant, particularly during the feeding regime, and/or feeding the ruminant with the ruminant feed composition according to the present disclosure.

The disclosure as defined here above will now be illustrated and explained in more detail in the following experimental part, which is not intended to limit the scope of this disclosure anyway.

Examples

## Example 1

Each NPN-source has specific characteristics, determining the release of N after entrance into the rumen. The efficiency of N-use by the rumen microbiota will however depend on the amount and the release rate of the NPN-source. This may result in a difference in the efficiency of microbial protein production in the rumen. In this example, the N-utilization *in vitro* of 3 different NPN-products and the mixture according to the present disclosure compared to coated and uncoated urea as NPN-sources for rumen fermentation was assessed.

### Test materials

A total of 6 different NPN-products and a mixture according to the present disclosure were selected to be tested in an *in vitro* rumen fermentation model (see Table 1) below. The mixture according to the present disclosure, in the different examples typically referred to by the code UB, generally comprises between 37 and 52 wt.% of urea, between 39 and 46 wt.% of biuret, between 3 and 6 wt.% of triuret, between 0.5 and 3 wt.% of ammelide, between 3 and 10 wt.% of cyanuric acid, and between 0.1 and 1.5 wt.% of moisture, based on the total weight of the mixture.

*Table 1: Overview of tested products, their supplier, physical form, purity and nitrogen content*

| Product | Supplied by | Physical form | Purity (%) | Nitrogen content (%) |
|---|---|---|---|---|
| Uncoated urea | Yara | Pearls | 99 | 46.2 |
| Urea | Yara | Pearls | 89 | 41.5 |
| Biuret | Alfa Aesar | Powder | 97 | 39.5 |
| Cyanuric acid | Yara | Powder | 100 | 32.6 |
| Triuret | Enamine | Powder | 95 | 36.4 |
| Ammelide | Dr. Ehrenstorfer | Powder | 99 | 43.3 |
| Mixture according to the present disclosure | Applicant | Powder | 100 | 41.0 |

### In vitro protocol

The *in vitro* model used to simulate rumen digestion and utilization of the products is the Gas Production Test protocol, adapted for protein digestibility, as described in more detail in the following paragraph.

Approximately 1 liter of rumen fluid was collected from two different rumen-fistulated animals in the morning. Rumen fluid was strained through a cheesecloth, kept at 39°C under $CO_2$ and then diluted 1:19 with a nitrogen-free buffer and mineral solution containing 10.03 g $NaHCO_3$, 1.43 g $Na_2HPO_4$, 1.55 g $KH_2PO_4$, 0.15 g $MgSO_4.7H_2O$, 0.52 g $Na_2S$, 0.017 g $CaCl_2.2H_2O$, 0.015 g $MnCl_2.4H_2O$, 0.002 g $CoCl_3.6H_2O$, 0.012 g $FeCl_3.6H_2O$ and 0.125 mg resazurin per liter.

To ensure the rumen fluid was depleted of free nitrogen and to ensure nitrogen would be the limiting factor for the microbial growth at the start of the experiment, the buffered rumen fluid was pre-incubated for 4 hours at 39°C with a carbohydrate overload of glucose, xylose and soluble starch, each at 3.33 g/l. During this pre-incubation, all nitrogen available in the rumen fluid would be incorporated into bacterial protein and other bacterial nitrogen-containing components.

Each product was weighed into different glass bottles on an isonitrogenous base, providing 15 mg N per flask. Then, 60 ml of the pre-incubated buffered rumen fluid was added, under continuous flushing with $CO_2$. Flasks were closed with rubber stoppers and placed in a shaking water bath with 50 movements per minute at 39 °C to start the incubation period. The gas production of each flask was recorded continuously for 72 hours using a fully automated system. All test products including a blank (i.e. no product added to the flask of rumen fluid) were tested in one run in triplicate.

The cumulative gas production was measured continuously for 72 hours. The results averaged per triplicate are shown in Figure 1.

*Results*

As can be seen in Figure 1, the accumulated gas production curves of ammelide and cyanuric acid were comparable to the blank sample. In general, the products are relatively low soluble in water compared to the other tested products. Moreover, the nitrogen in these products is incorporated in a ring structure which is less accessible. The nitrogen solubility of ammelide and cyanuric acid appeared to be too low to support bacterial growth. For ammelide, cyanuric acid as well as the blank, the gas production is however not equal to zero since microbial turnover release small amounts of nitrogen into the solution which can be used by the remaining microorganisms. The cumulative gas production after 72 hours was not significantly different between the blank, ammelide and cyanuric acid.

As can furthermore be seen in Figure 1, the cumulative gas production profiles of the uncoated urea and the coated urea showed a lag period of approximately 5 hours, after which the cumulative gas production rapidly increased to a maximum of 17 ml/hour for the uncoated urea and 19 ml/hour for the coated urea. After approximately 15 hours of incubation, the cumulative gas production slowed down rapidly and stopped after 30 hours. The solubility of urea in water has been shown to be very quick, within minutes. The longer lag time of 5 hours for urea products can be explained from the effect of the fast release of ammonia on the pre-incubated buffered rumen fluid, preventing the release of $CO_2$ from the fluid. Coated urea was expected to have a slower nitrogen release rate than uncoated urea, which did not result in a difference in cumulative gas production over the 72 hours of incubation. The cumulative gas production of the uncoated urea was numerically lower than the coated urea between 10 and 20 hours of incubation, which may be related to the slower release of the nitrogen from the coated urea. Biuret and triuret showed a cumulative gas production curve below the coated and uncoated urea. Biuret had a comparable lag time of 5 hours, but a slower gas production rate in the first 15 hours of incubation. The cumulative gas production was the highest in the period of between 15 and 25 hours of incubation, and slowed down afterwards until the maximum cumulative gas production was reached after 40 hours. Triuret showed a longer lag time of 9 hours, suggesting a slower release of nitrogen which was expected based on the characteristics of triuret relative to biuret which is less soluble. After 9 hours, the gas production rate started relatively slowly, peaking at 20 hours of incubation. The cumulative gas production was rather stable at a low level of approximately 4 ml/hour from 20 to 50 hours and decreased afterwards until the end level was reached after 60 hours.

As further can be seen in Figure 1, the cumulative gas production of the mixture according to the present disclosure was higher in comparison with coated urea during the 72 hours of incubation. In the period between 20 and 40 hours of incubation, the cumulative gas production for the mixture according to the present disclosure remained lower than the cumulative gas production of coated urea, which is likely related to the slower release of N from the biuret and other NPN compounds present in this mixture. Nevertheless, the cumulative gas production from the mixture according to the present disclosure kept increasing after 40 h, obtaining the greatest cumulative gas production amongst all the NPN sources tested.

*Conclusion*

The rapid ammonia release rate of uncoated urea limits the amount of urea that may be used in a ruminant diet formulation. High inclusion levels of uncoated urea in a ruminant diet formulation will increase the ammonia concentration in the rumen rapidly, leading to ruminal accumulation and absorption of ammonia and subsequent excretion of urea in the urine.

As expected, the pure N-compounds, i.e. ammelide, triuret and cyanuric acid, which are well-known N-containing by-products of the biuret production by thermal decomposition of urea and which are also present in minor amounts in the mixture according to the present disclosure, generated a much lower cumulated gas production than uncoated and coated urea. Ammelide and cyanuric seem not to be suitable as rumen digestible nitrogen sources in dairy cattle nutrition. Their solubility and nitrogen release rate are too slow to support microbial growth. Nitrogen from biuret and triuret can be used by rumen bacteria, but it is released more slowly than coated or uncoated urea. The slow release of nitrogen from triuret and biuret may improve nitrogen utilization. However, it can be expected to escape from the rumen approximately 12 to 24 hours after ingestion (depending on the diet characteristics such as digestibility and particle size). Part of the biuret and triuret (approximately 60%) will still be unused by the rumen microbes after 24 hours, based on the cumulative gas production profiles. This amount of unused nitrogen is likely to pass the rumen and be excreted after passage through the gastrointestinal tract.

The mixture according to the present disclosure, particularly the proportion of urea, biuret and the minor N-compounds created by the production of biuret starting from urea therein, resulted in an unexpected fermentation pattern as shown by the cumulative gas production dynamics, reaching the greatest total gas production amongst all the NPN sources assessed. As can be seen in Figure 1, in the beginning, the slope of the cumulative gas production curve is less steep in comparison with the one of urea and uncoated urea, presumably due to a lower amount of rapidly available urea, but afterwards, when the more slowly fermented biuret is used by the microorganisms, the cumulative gas production is the highest of all tested products. The more cumulative gas is produced, the better, since this means that rumen fermentation is maximized. This indicates that the mixture according to the present disclosure, comprising urea, biuret and N-containing by-products of the biuret production out of urea has a synergistic effect on the rumen fermentation compared to the fermentation of the individual components (taken individually).

## Example 2

In this example, an *in vitro* assay was performed using dual-flow continuous culture fermenters, aiming to simulate rumen fermentation.

*Fermenters*

Eight 1320 ml dual-flow continuous culture fermenters were used in two replicated periods. Each period consisted out of 5 days for adaptation of the ruminal fluid to the treatments and 3 days for sampling. On the first day of each period, all fermenters were inoculated with undiluted ruminal fluid filtered through two layers of cheesecloth to remove the feed particles. The fermentation conditions were constant, i.e.

- a temperature of 39 °C,
- a pH of 6.4 $\pm$ 0.05 by infusion of 3 N HCl or 5 N NaOH,
- anaerobiosis with continued infusion of $N_2$ gas at a rate of 40 ml/min, and
- continued infusion of artificial saliva to obtain liquid and solid dilution rates of 10 %/h and 5 %/h, respectively.

The fermenters were fed 95 grams/day of dry matter of a diet formulated to meet or exceed current nutrients recommendations for lactating dairy cows, i.e.

- 166 g/kg crude protein (CP),
- 323 g/kg neutral detergent fiber (NDF), and
- 201 g/kg acid detergent fiber (ADF),

And were fed in three daily administrations, i.e. at 7, 15 and 23 o'clock with a forage : concentrate diet in a range of 53 : 47.

## *Animals and treatment diets*

Ruminal fluid was taken from two Holstein dairy cows fitted with ruminal cannulas fed with a 60 : 40 forage : concentrate diet with alfalfa and cracked grain (11 kg dry matter/cow/day). The afternoon before the ruminal extraction, the ruminant animals were not fed.

The NPN-dietary treatments were as follows:

- Control: basal diet to which 0.51 wt.% urea was added, with wt.% on dry matter basis,
- Diet: basal diet to which 0.55 wt.% of the mixture according to the present disclosure, with composition as in example 1, was added, with wt.% on dry matter basis.

To obtain isoenergetic and isonitrogenous diets using different NPN sources, a nitrogen content determination using the Kjeldahl method was used to determine the nitrogen content of each NPN product (see Table 2 below).

*Table 2: Nitrogen content of non-protein nitrogen sources*

| Experimental example | N wt.% |
|---|---|
| Control | 44.37 |
| Diet | 40.94 |

Due to the N-percentage differences among the products, the inclusion level of each NPN-source was different (see Table 3 below).

*Table 3. Ingredient composition of the dietary treatments*

| Ingredient (wt.% of dry matter) | NPN dietary treatment | |
|---|---|---|
| | Control | D1 |
| Corn grain, craked dry | 40.6 | 40.6 |
| Legume hay < 40 wt.% NDF | 26.7 | 26.7 |
| Grass, hay, mature | 26.3 | 26.3 |
| Soybean meal, 48 wt.% CP | 4.61 | 4.61 |
| Urea | 0.51 | - |
| Mixture according to the present disclosure (see ex. 1) | - | 0.55 |
| Calcium carbonate ($CaCO_3$) | 0.46 | 0.46 |

### Sample collection

On the second day of sampling, 4 ml of filtered fermenter fluid was taken 2 hours after the morning feeding to determine VFA-concentrations.

During sampling days, the collection vessels were maintained at 4 °C to prevent microbial activity. Solid and liquid effluents from 3 sampling days were composited and mixed within the fermenter and homogenized. Subsamples were taken for the analyses of the volatile fatty acids (VFA). The remainder of the sample was lyophilized (freeze drying) and the dry samples were analyzed for DM, ash, N and purines to calculate bacterial nitrogen and organic matter content (OM).

Bacterial cells were obtained from the fermenter flasks the last day of each experimental period. Solid and liquid associated bacteria were isolated using a combination several detachment procedures selected to obtain the maximum detachment without affecting the cell integrity. The bacterial cells were lyophilized and the dry samples were analyzed for dry matter (DM), ash, N and purines to calculate bacterial nitrogen.

### Chemical analyses

Effluent dry matter (DM) was determined by lyophilizing 300 ml aliquots in triplicate. The DM content of the diets and bacterial samples were determined by drying samples for 24 hours in an 103 °C forced air oven (see AOAC, 1990; method 950.01).

Dry samples of diets, effluents and bacteria were ashed overnight at 550 °C in a muffle furnace (see AOAC, 1990, method 976.05) and organic matter (OM) was determined by difference. Total N of diets, effluents and bacterial samples were determined by a Kjeldhal method (AOAC, 1990; method 976.05). Sample crude protein (CP) was calculated as $N \times 6.25$. Effluent CP was determined in liquid samples. Crude protein degradation was calculated as the difference between CP in the diet minus CP in the effluent and expressed as wt.% of DM. The EMPS was determined by dividing bacterial N by fermented organic matter.

The fiber components of the diets and the effluents were analyzed sequentially using a thermostable alpha-amylase and sodium sulfite and expressed without residual ash.

The samples for the VFA-analysis were prepared as described by Jouany, J. P., and J. Senaud. "Influence des ciliés du rumen sur la digestion de différents glucides chez le mouton. I.-Utilisation des glucides pariétaux (cellulose et hémicelluloses) et de l'amidon." Reproduction Nutrition Développement 22.5 (1982): 735-752. and analyzed by gas chromatography.

*Results*

In Table 4 below, the effect of the NPN-diets on the nitrogen metabolism of ruminal microorganisms, true digestibility of DM and OM, CP degradation, the efficiency of microbial protein synthesis (EMPS) in the rumen (or in other words, how much microbial protein is produced due to the microbial activity for every unit of substrate which is fed to the ruminant), in the control diet ("Control" - comprising urea) and a diet ("Diet" - comprising UB) comprising a mixture according to the present disclosure is shown.

*Table 4: Effect of NPN dietary treatment on the nitrogen metabolism of ruminal microorganisms*

|  | NPN dietary treatment | |
| --- | --- | --- |
|  | Control (urea) | Diet (UB) |
| True digestibility, wt.% of DM |  |  |
| DM | 53.53 | 56.79 |
| OM | 52.95 | 55.90 |
| CP degradation (% of dry matter) | 47.7 | 53.2 |
| EMPS (g bacterial N/kg of fermented organic matter) | 35.7 | 39.4 |
| VFA |  |  |
| Acetate | 62.92 | 60.06 |
| Propionate | 18.18 | 23.29 |
| Acetate to propionate ratio | 3.5 | 2.76 |

*Conclusion*

Out of Table 4, it can be concluded that the diet comprising a mixture of urea, biuret and the minor amount of N-containing compounds originating from the biuret production out of urea according to the present disclosure have

- a higher true digestibility of DM and OM, with an increase in digestibility of about 5%-6% compared to a diet comprising urea as NPN source,
- an increase of the EMPS, meaning that the rumen microbiota maximized N-utilization, with an increase of EMPS of about 10% compared to a diet comprising urea as NPN source, and
- an increased amount of propionate and a decreased amount of acetate, through which acetate to propionate ratio is decreased, suggesting that this NPN-mixture according to the present disclosure improves the efficiency of the ruminal energy utilization, with a decrease in acetate to propionate ratio of about 20% compared to a diet comprising urea as a NPN source.

## Example 3

*Animals, experimental design and treatments*

A total of 72 crossbred yearling steers [303 kg $\pm$ 29 kg initial body weight (BW) were stratified by body weight (BW) and randomly allocated to three treatments. All steers consumed a basal diet of corn silage, cotton-gin trash, and a premix of vitamins and minerals.

The steers were housed in pens (8 steers / pen, 3 pens / treatment). The steers were supplemented with two different NPN-sources on an isonitrogenous basis, considering as reference the N provided by urea when included at 1 wt.% of the diet in dry matter (DM) basis.

In Table 5, the composition of the basal diet supplemented with two different types of NPN, i.e. urea (Urea) (46 % N) and a mixture of urea and biuret according to the present disclosure (UB), with composition as in example 1 (41 % N) is shown.

In order to determine DM of the feed, approximately 0.5 g of sample was weighed in duplicate, dried in a forced-air oven at 100 °C for 24 hours, and ashed at 550 °C for six hours.

For the determination of the fibrous component, samples were weighed in duplicate into F56 bags (Ankom Technology Crop., Macedon, NY) and analyzed for neutral detergent fiber (NDF), using heat-stable $\alpha$-amylase and sodium sulfite, and subsequently for acid detergent fiber (ADF) as described by Van Soest et al. (1991) in Ankom 200 Fiber Analyzer (Ankom Technology Corp., Macedon, NY).

The concentration of N was analyzed through the Dumas dry combustion method using a Vario Micro Cube (Elementar, Manchester, UK) after samples were ball-milled using a Mixer Mill MM 400 (Retsch) at 25 Hz for 9 minutes.

The feed samples were analyzed for concentration of starch through the enzymatic-colorimetric method of Hall (2015) with the following modifications: glucose was analyzed using a quantitative colorimetric kit (G7521-11, Pointe Scientific Inc., Canton, MI), absorbance was read on 200 $\mu$l samples at OD520 in flat-bottom 96-well plates (Corning Costar 3361, Thermo Fisher Scientific Inc., Waltham, MA) using a plate reader (Fisherbrand UV / VIS AccuSkan GO Spectrophotometer, Thermo Fisher Scientific Inc., Hampton, NH).

*Table 5: Composition of the basal diet supplemented with different types of NPN*

| Item | Treatment | |
|---|---|---|
| | +Urea | +UB |
| Ingredient, wt.% of dry matter | | |
| Corn silage | 77.00 | 76.88 |
| Cotton-gin trash | 20.00 | 20.00 |
| Mineral | 2.00 | 2.00 |
| NPN source | 1.00 | 1.12 |
| Composition | | |
| DM (dry matter), wt.% as fed | 37.13 | 36.17 |
| CP (crude protein), wt. % of DM | 13.20 | 12.70 |
| Soluble CP, wt. % of CP | 49.00 | 53.00 |
| iNDF (indigestible neutral detergent fiber) | 41.40 | 38.30 |
| ADF (acid detergent fiber) | 30.10 | 29.40 |
| Starch, wt.% of DM | 27.70 | 29.80 |
| Ash, wt.% of DM | 5.91 | 5.78 |

***Animal adaptation***

From day -17 to day -16 relative to the beginning of the experimental period, the steers received 20 % of their total supplemental NPN.

From day -15 to day -12 relative to the beginning of the experimental period, the steers received 40 % of their total supplemental NPN.

From day -11 to day -8 relative to the beginning of the experimental period, the steers received 60% of their total supplemental NPN.

From day -7 to day -3 relative to the beginning of the experimental period, the steers received 80 % of their total supplemental NPN.

From day -2 to the beginning of the experimental period (day 0), the steers received 100% of their total supplemental NPN.

***Blood profile measurements***

Blood samples were collected every 14 days during the performance evaluation via jugular venipuncture into 10 ml evacuated tubes containing Na heparin (BD Vacutainer, Franklin Lakes, NJ) and placed on ice centrifuged at 1,500 x g for 15 minutes at 4 °C. Plasma was transferred to polypropylene tubes (12 x 75 mm, Fisherbrand, Thermo Fisher Scientific Inc, Waltham, MA) and stored at - 20 °C for further analysis.

***Enteric methane emission measurements***

When feed is eaten by a ruminant, the nutrients are initially in the form of carbohydrates, proteins and fats (or lipids). During the fermentation of the carbohydrates, carbon dioxide and methane are produced.

Methane emission was measured in all steers for five consecutive days using the $SF_6$ tracer technique (Johnson

et al., 1994; Henry et al., 2020). In this technique, the steers received a calibrated permeation tube filled with $SF_6$ (3.1 g $\pm$ 0.2 g) before the measurement period. The average $SF_6$ release rate was 5.2 g $\pm$ 1.1 mg/d. After five days of training, a vacuum gas collector canister was attached to each animal to collect a sample of daily gas production. Each day at the same hour, a new vacuum canister was replaced throughout the five days of the measurement period. Five collection canisters were used to determine environmental $CH_4$ and $SF_6$ concentrations. A gas sample was taken from each canister and conserved in 120 ml bottles for posterior analysis of $CH_4$ and $SF_6$ using gas chromatography. For the current experiment, only steers with a least three successful days of collection and measurement were considered in the final analysis. Gas samples were analyzed by gas chromatography (Agilent 7820A GC, Agilent Technologies, Palo Alto, CA). A flame ionization and electron capture detector were used for $CH_4$ and $SF_6$ analysis, respectively, with a capillary column (Plot Fused Silica 25 m x 0.32 mm, Coating Molsieve 5A, Varian CP7536, Varian Inc., Lake Forest, CA). The injector, column and detector temperatures for $CH_4$ analysis were 80, 160 and 200 °C, respectively. For $SF_6$, temperatures were 50, 30 and 300 °C for the injector, the column and the detector, respectively. The carrier gas for $CH_4$ and $SF_6$ was $N_2$. The emissions of $CH_4$ were calculated according to the next equation:

$$QCH_4 = QSF_6 \; x \frac{([CH_4]\gamma - [CH_4]\beta)}{([SF_6]\gamma - [SF_6]\beta)}$$

$QCH_4$ = $CH_4$ emissions per animal (g / day)
$QSF_6$ = $SF_6$ release rate (mg / day)
$[CH_4]\gamma$ = the concentration of $CH_4$ in the animals' collection canister
$[CH_4]\beta$ = the concentration of $CH_4$ in the environmental collection canister
$[SF_6]\gamma$ = the concentration of $SF_6$ in the animals' collection canister
$[SF_6]\beta$ = the concentration of $SF_6$ in the environmental collection canister

### *Gain to feed ratio*

The gain to feed ratio (G:F) was computed as the ratio of the average daily gain (ADG) to daily dry matter intake (DMI). The feed intake was recorded daily. The individual intake was determined via the GrowSafe® intake monitoring system. In cattle management, GrowSafe Systems is a commonly used tool to closely monitor individual animal feeding data using radio frequency identification (RFID) tag technology. The animals are equipped with a unique RFID tag that is read by the feed bunks each time the animal lowers its head into the bunk to consume feed. Average daily weight gain (ADG) and feed efficiency were calculated during the performance evaluation. The ADG was determined by the difference between the final BW and the initial BW divided by the number of days of the performance evaluation (56 days). The initial BW was calculated as the average unshrunk BW of heifers on days 16 and 17. The final BW was the average unshrunk BW measured on days 72 and 73. The unshrunk BW was obtained every 14 days.

### *Effect of NPN supplementation on animal performance and plasma urea nitrogen (PUN)*

In Table 6, the effect of NPN supplementation with Urea and UB on growth performance is shown. More in particular, the initial and the final BW and the ADG, all expressed in kg, the dry matter intake (DMI) expressed in kg / day and in percentage of BW, the gain to feed ratio (G:F) expressed in kg / kg, the net energy of gain (NEg) expressed in Mcal / kg of DM and the plasma urea nitrogen (PUN) expressed in mg / dL are shown.

*Table 6: Effect of NPN supplementation on animal performance and the plasma urea nitrogen (PUN)*

| Item | Treatment | |
|---|---|---|
| | +Urea | +UB |
| Initial BW (kg) | 288.69 | 283.18 |
| Final BW (kg) | 343.69 | 342.11 |
| ADG (kg) | | |
| 0-14 | 0.78 | 0.72 |
| 0-28 | 0.81 | 0.72 |
| 0-42 | 1.02 | 1.04 |
| 0-56 | 1.01 | 1.05 |

(continued)

| Item | Treatment | |
|---|---|---|
| | +Urea | +UB |
| DMI (kg / day) | | |
| 0-14 | 6.82 | 6.91 |
| 0-28 | 7.03 | 7.40 |
| 0-42 | 7.24 | 7.85 |
| 0-56 | 7.29 | 7.98 |
| DMI (% of BW) | 2.31 | 2.54 |
| G:F (kg / kg) | | |
| 0-14 | 0.10 | 0.10 |
| 0-28 | 0.11 | 0.10 |
| 0-42 | 0.14 | 0.13 |
| 0-56 | 0.14 | 0.13 |
| NEg (Mcal / kg of DM) | 0.55 | 0.52 |
| PUN (mg / dL) | | |
| 0 | 7.80 | 7.59 |
| 14 | 6.76 | 7.52 |
| 28 | 10.41 | 8.78 |
| 42 | 9.25 | 7.55 |
| 56 | 8.20 | 8.60 |

Table 6 demonstrates that, compared to supplementation with urea, supplementation with the UB NPN source:

- increased ADG, particularly during the second half of the test period;
- increased DMI during the whole duration of the experiment, with an increase in DMI of at least 5% during the different time periods considered, compared to the diet containing urea as NPN source;
- increased DMI per unit of BW, with an increase of about 10% compared to the diet containing urea as NPN source.

### *Effect of NPN supplementation on enteric emissions of beef steers*

Methane is an energy loss representing between 2 % and 12 % of the gross energy intake in ruminants.

In Table 7, the effect of the NPN supplementation with Urea and UB on enteric methane emission of beef steers is shown. More in particular, the intake expressed in kg / day, and the methane emission expressed in g / kg DMI, g / kg OMI and g/ kg ADG are shown.

*Table 7: Effect of NPN supplementation on enteric methane emission of beef steers*

| Item | Treatment | |
|---|---|---|
| | +Urea | +UB |
| DM intake (kg / day) | 6.08 | 7.11 |
| Methane emission | | |
| (g / kg DMI) | 36.59 | 31.21 |
| (g / kg OMI) | 38.68 | 32.94 |
| (g / kg ADG) | 213.79 | 212.31 |

Out of Table 7, it can be concluded that supplementing the diet of yearling steers with UB reduced methane emission without compromising or even improving their performance. In particular, a decrease in methane emissions, when expressed as g/kg DMI or g/kg OMI, of about 15% was observed, compared to a diet containing urea as NPN source. Simultaneously, it was observed that the DMI considered over the whole duration of the experiment was increased by about 17% when using UB as NPN source, compared to the diet containing urea as NPN source.

### Example 4

In this example, an *in vitro* assay was performed using batch culture incubations, aiming to simulate rumen fermentation.

### *Animal adaptation*

Two ruminally cannulated Angus crossbred steers (808.8 $\pm$ 36.3 kg body weight) were used as ruminal fluid donors for the in vitro batch culture incubations. The steers were fed a corn silage and gin trash diet (70% and 30% on a dry matter basis, respectively) ad libitum at least 35 days before collecting ruminal fluid to perform the in vitro incubations. Each steer received a NPN-mixture equivalent to 100 g of urea per day comprised of an equal amount of N from the different NPN sources to adapt the rumen microbial community. Thus, each steer was fed daily 33 g of urea (46 wt.% N) or 37 g of a urea-biuret mixture according to the present disclosure (UB) comprising 41 wt.% N. These amounts were mixed in with the feed diet every two days.

### *Feed preparation and chemical analysis*

The feed was prepared by drying corn silage and gin trash for 45 hours at 55°C and ground to pass a 2 mm screen in a Wiley mill.

### *Experimental design and treatments*

The in vitro batch culture incubations were conducted as a randomized complete block design using 70 wt.% corn silage and 30 wt.% gin trash on a dry matter basis conserving the diet characteristics used during the animal adaptation period. The treatments were designed to be isonitrogenous (= having the same amount of dietary nitrogen) and equivalent to 1 wt.% of urea inclusion. The NPN treatments were consequently as follows:

- Control without NPN-supplementation ("control");
- 100 wt% urea (Rumisan ®) ("urea");
- 100 wt% of a urea/biuret mixture according to the present application ("UB").

### *Rumen fluid collection and in vitro incubations*

The *in vitro* incubations were conducted on three separate days (replicates). A representative sample of digesta was collected from different places in the rumen of ruminally cannulated steers and strained through 4 layers of cheesecloth, placed in pre-warmed thermos containers, and transported to the laboratory within 30 minutes after the collection. In the laboratory, the ruminal fluid from the two steers was maintained under constant $CO_2$-flux and was combined in equal proportions. This combined ruminal fluid was mixed with McDougall's buffer (McDougall, 1948) with and without 0.077 g/liter of 10 wt.% ammonium sulfate enriched with $^{15}$N in relation 1:4, and it was used as an inoculum. Two bottles of 500 ml fitted with a side arm and a rubber septum were incubated per treatment. The bottles contained 5.6 g of substrate dry matter and 400 ml of $^{15}$N enriched inoculum, and were incubated for 24 hours at 39°C with an agitation of 60 rpm to monitor gas production kinetics using an Ankom Gas Monitoring System from Ankom Technologies, Fairport, New York. Also, one 500-ml bottle without $^{15}$N enriched inoculum was incubated to determine the basal $^{15}$N enrichment. At 12 hours of incubation, two samples of 10 ml of rumen fluid were collected from each bottle through the septa port using a 20-ml syringe. These samples were acidified by adding 10 $\mu$l of a 20 vol/vol% of $H_2SO_4$-solution to each 10-ml sample, and frozen at minus 20°C until further analysis. At the end of the 24 hours incubation, the final pH was recorded, the bottles were placed on ice to prevent further microbial fermentation and two 10-ml samples were collected and acidified by adding 20 $\mu$l of a 20 vol/vol% of $H_2SO_4$-solution and were frozen at minus 20°C until further analysis. The remaining fermentation content was thereafter agitated at 60 rpm for 30 seconds and divided into two subsamples. From the first subsample, composed of whole contents, a volume of 180 ml was frozen at minus 20°C until further analysis and from the second subsample 180 ml were used to recover the microbial pellet. Furthermore, two tubes of 100 ml per treatment containing 0.7 g of the substrate on a dry matter basis and 50 ml of inoculum without $^{15}$N enrichment and two blanks were incubated for 24 hours at 39 °C with agitation at a speed of 60 rpm. After the 24 hours incubation with ruminal fluid, 6 ml of HCl and 2 ml of a 5% pepsin solution were added. The tubes were incubated for another 48 hours at 39°C. After the incubation period, the tubes were maintained on ice and later stored for further analysis.

*Concentrations of volatile fatty acids (VFA)*

VFA produced by microbial fermentation in the rumen are the main source of energy absorbed by the digestive tract of ruminants. The amount and the profile of VFA in the rumen has consequences for the efficiency of energy utilization, production of methane, risks of ruminal acidosis (characterized by a decrease in pH caused by an accumulation of organic acids in the rumen and a shift in the delicate balance of rumen microorganisms). A water-based solution using ethyl acetate extraction was used to determine VFA concentrations at 12 and 24 hours. Samples were centrifuged at $10,000 \times g$ for 15 min at 4°C, 2 ml of the supernatant were mixed with 0.4 ml (5:1 ratio) of a metaphosphoric-crotonic acid solution, and samples were frozen overnight. Samples were then thawed and centrifuged again at $10,000 \times g$ for 15 min at 4°C. The supernatant was transferred into $12 \times 75$ mm borosilicate disposable culture tubes (Fisherbrand; Thermo Fisher Scientific Inc., Waltham, MA) and mixed with ethyl acetate to form a 2:1 ethyl acetate : supernatant mixture. The samples were analyzed with a gas chromatograph (Agilent 7820A GC, Agilent Technologies) using a flame ionization detector and a capillary column (CP-WAX 58 FFAP 25 m $\times$ 0.53 mm, Varian CP7767; Varian Inc.).

*Microbial protein synthesis*

The rumen microbial ecosystem is made up mainly by species of bacteria, fungi, and protozoa. The microbes ferment ingested feeds by the ruminant, and, by fermentation, produce end products that are utilized by the ruminant as well as by the microbes themselves for their own proliferation and growth. The species diversity and their relative proportions within the microbial community depend primarily on the diet consumed by the animal. It is thus important that a healthy mixture of different feedstuffs is fed to the animal to keep the rumen and its microflora functioning efficiently. The speed of digestion of the feed depends on the quality and the supplement of the feed and is affected by the number and type of microbes, the pH in the rumen, the nutrients limiting the growth of the microbes and the removal of microbes from the rumen. Energy and protein are the major nutrients that limit the microbial growth, and therefore, the rumen fermentation. The microbial population needs energy and protein for their growth and multiplication. Protein, when digested, is broken down into peptides, which are short chains of amino acids. Further digestion of peptides results in individual amino acids and eventually ammonia. Rumen microbes are the major source of protein for the ruminant animal. If there is enough energy available, the rumen microbes break down the rumen degradable protein to amino acids and then to ammonia which is a major N-source for microbial growth. The microbes also convert NPN to ammonia, which contributes to the ammonia pool available for microbial growth. Microbial protein is continuously flushed from the rumen into the intestine where undergoes enzymatic digestion. The amino acids produced from the digested microbial protein are absorbed through the small intestine. The amount of microbial protein flowing into the intestines therefore largely depends on the availability of fermentable energy and N in the diet consumed by ruminants.

The total nitrogen (N) and the isotopic supplement were measured in the whole content and the bacterial pellet by an isotopic ratio mass spectrometry using a Vario Micro cube from Elementar Analyzer System GmbH. The bacteria pellet subsample was slowly centrifuged at 1,000 x g for 10 minutes at 4°C. The resulting supernatant was fast centrifuged at 20,000 x g for 20 minutes at 4°C to obtain the pellet of bacteria. This bacteria pellet was then washed with a 0.9% saline solution and centrifuged at 20,000 x g for 20 minutes at 4°C, discarding the supernatant. The saline wash procedure was repeated twice. Finally, the bacterial pellet was freeze-dried. Separately, the 180-ml whole content subsample was completely freeze-dried. The dried samples of the whole content and of the bacterial pellet were manually grinded and weighted into an 8 x 5-mm pressed standard-weight tin capsule using a Mettler-Toledo Excellence Plus XP Micro Balance from Mettler-Toledo GmbH, Laboratory and Weighing Technologies. Then, 35 $\mu$l of a 10 g/l solution of $K_2CO_3$ were added to each sample and dried overnight in a forced-air oven at 60°C for complete $NH_3$-N evaporation. The percentage of atom $^{15}N$ in the dried samples was analyzed in an isotope ratio mass spectrometer (IsoPrime 100 from IsoPrime) and expressed as the fractional abundance of isotopic fractions ($^{15}N$ / $^{14}N$) multiplied by 10.

*Concentration of ammonia-N*

Ruminal ammonia production largely reflects the amount of degradable protein in the rumen. Ruminal ammonia nitrogen (ammonia-N) is a critical nutrient for supporting microbial growth and fermentation. Ensuring the availability of ammonia-N in the rumen both on a quantitative and timely manner is fundamental for achieving maximum rate of fermentation and feed digestibility.

The concentration of ammonia-N at 12 and 24 hours in the incubation fluid was measured after centrifuging at 10,000 x g for 15 minutes at 4°C using an Avanti J-E centrifuge from Beckman Coulter® Incorporated. One ml of phenol reagent was pipetted into $12 \times 75$ mm borosilicate disposable culture tubes (Fisherbrand; Thermo Fisher Scientific Inc.,

Waltham, MA). A 20-µl aliquot of the supernatant from the centrifuged sample was transferred to the phenol-containing culture tubes. After vortexing, 0.8 ml of hypochlorite solution was added to the mixture and was vortexed again. The culture tubes were covered with glass marbles and placed in a water bath at 95°C for 5 minutes. The absorbance was read in 96-well, flat-bottom plates at a wavelength of 665 nm using a DU-500 plate reader from Beckman Coulter® Inc. The fermentation residue reserved into the tubes of 100 ml were filtered, dried at 105°C in a forced air oven for 24 hours and ashed at 550°C for 6 hours to determine the undigested organic matter.

### Nutrient disappearance

Digestibility represents the proportion of ingested nutrients consumed that are absorbed by the animal. Dry matter is defined as the loss of weight of samples when dried in an oven above 100°C during 12 - 24 hours, whereas organic matter defines the weight loss of the dry matter when combusted (the dry matter minus the ash content). Dry matter consists of all nutrients, whereas organic matter consists of all nutrients with the exception of ash. Organic matter digestibility is defined as the proportion of organic matter in the feed that is digested in the rumen or total ruminant digestive tract. Ruminal organic matter digestibility can be used to estimate energy available for fueling microbial growth and the resulting synthesis of microbial protein in the rumen.

The in vitro dry organic matter digestibility (IVDMD) and the in vitro organic matter digestibility (IVOMD) was calculated with the following formulae:

$$IVDMD\ (\%) = \frac{incubated\ dry\ matter - residual\ dry\ matter}{incubated\ dry\ matter}\ x\ 100$$

$$IVOMD\ (\%) = \frac{incubated\ organic\ matter - residual\ organic\ matter}{incubated\ organic\ matter}\ x\ 100$$

### Results

In Table 8 below, the concentration of the total VFA and the concentration of ammonia-N in: a control diet without NPN supplementation ("control"), a corn silage-based diet either with urea supplementation ("urea") or with supplementation of a urea-biuret mixture according to the present disclosure ("UB") after 12 hours of incubation is shown. In Table 9 below, the same is shown after 24 hours of incubation. In Table 9, also the pH of the fluid, the *in vitro* dry matter degradability (IVDMD) and the *in vitro* organic matter degradability (IVOMD) after 24 hours of incubation are shown. The pH of the rumen has profound effects on the growth of the ruminal microbes and the digestion that takes place in this forestomach. Rumen pH can affect the extent of rumen fermentation by altering microbial populations and tissue absorptive capacity. Low ruminal pH for prolonged periods each day for instance can affect feed intake, microbial metabolism and feed digestion, and has also been related to inflammation, diarrhea and milk fat depression.

*Table 8: Effect of NPN supplementation on VFA and ammonia-N at 12 hours of incubation in a corn silage-based diet*

| Variable | Control | Urea | UB |
|---|---|---|---|
| | mM | | |
| Total VFA | 30.95 | 31.77 | 32.55 |
| Ammonia-N | 0.35 | 2.74 | 2.61 |

*Table 9: Effect of NPN supplementation on VFA and ammonia-N, pH and in vitro digestibility or organic and dry matter at 24 hours of incubation in a corn silage-based diet*

| Variable | Control | Urea | UB |
|---|---|---|---|
| | mM | | |
| Total VFA | 41.39 | 40.96 | 42.85 |
| Ammonia-N | 2.48 | 6.55 | 7.34 |
| pH | 6.34 | 6.38 | 6.37 |
| IVDMD, % | 45.02 | 45.08 | 45.78 |

(continued)

| Variable | Control | Urea | UB |
|---|---|---|---|
| IVOMD, % | 64.13 | 65.38 | 64.17 |

Out of Tables 8 and 9, it can be concluded that

- With regards to the total VFA, the UB mixture according to the present application generated a higher amount of VFA in comparison with the control without NPN supplementation or with urea supplementation. These findings indicate that the mixture of urea and biuret according to the present disclosure is more effective in supporting in vitro fermentation than urea supplementation.
- With regards to ammonia-N, as expected, the supplementation with NPN, i.e. urea or the UB mixture increased its concentration.
- With regards to IVDMD, supplementation with UB, but not with urea, improved the extent of dry matter degradation. These findings further support the proposition that supplementation with UB is more effective in supporting rumen fermentation than urea.
- Overall, these results demonstrate that the UB mixture according to the present application is an effective source of NPN that outperforms urea in supporting in vitro fermentation.

In Table 10, the concentration of bacterial N, bacteria efficiency (BE) or, stated differently, the efficiency of microbial protein synthesis (EMPS), i.e. g of bacteria N / kg of OM truly digested for (i) a control diet without NPN supplementation ("control"), (ii) a corn silage-based diet with urea supplementation ("urea") or (iii) a corn silage-based diet with supplementation with a urea-biuret mixture according to the present application ("UB"), after 24 hours of incubation is shown. These parameters define the capacity of rumen microbes to use N.

*Table 10: Effect of NPN supplementation on microbial synthesis and microbial efficiency production in a corn-based diet*

| Variable | Control | Urea | UB |
|---|---|---|---|
| Bacteria N, mg | 55.66 | 67.34 | 59.55 |
| EMPS | 27.07 | 34.46 | 41.98 |

Out of Table 10, it can be concluded that the supplementation of the control diet with NPN increases the amount of N assimilated by bacteria. Supplementation with the urea/biuret mixture according to the present application particularly resulted in an increased bacteria efficiency/increased efficiency of microbial protein synthesis. These results are in line with the results of example 2.

### Methane production

When feed is eaten by a ruminant, the nutrients are initially in the form of carbohydrates, proteins and fats (or lipids). During the fermentation of the carbohydrates, carbon dioxide and methane are produced.

The amount of methane produced was determined by measuring total gas production and the contribution of methane to it. The production of gas was recorded using an Ankom Gas Monitoring System from Ankom Technologies. This system was adapted to 500 ml bottles during the entire fermentation process. The kinetics of the production was fitted to a Gompertz model to determine the rate of the total maximal gas production (M), fractional rate of gas produced (kf) and lag phase (L). A gas subsample was analyzed to measure methane concentration and calculate the total methane production during the fermentation. The concentration of methane was determined using a gas chromatograph Agilent 7820 GC from Agilent Technologies with flame ionization and a capillary column (Plot Fused Silica 25 m x 0.32 mm, coating Molsieve 5A, Varian CP 7536 from Varian Inc.).

In Table 11, the effect of NPN supplementation on the production of methane in a corn silage-based diet is shown. More in particular, methane gas production expressed in mM/g organic matter incubated (OMi) and expressed in mM/g organic matter digested (OMd), in a control diet without NPN supplementation ("control"), a corn silage-based diet either urea supplementation ("urea") or with a urea/biuret mixture according to the present application ("UB"), are shown.

Table 11 shows that supplementation with a UB mixture according to the present disclosure resulted in a decreased production of methane compared to the control diet or the diet with urea as NPN. Methane production decreased by about 4-5% when expressed per g of OMi or per g of OMd, compared to urea as NPN.

Table 11: Effect of NPN supplementation on methane production in a corn silage-based diet

| Variable | Control | Urea | UB |
|----------|---------|------|-----|
| mM/g OMi | 1.24 | 1.23 | 1.18 |
| mM/g OMd | 6.93 | 6.86 | 6.58 |

## Example 5 - increasing doses of UB on fermentation and methane production *in vitro*

The main objective of example 5 was to assess the impact of increasing doses of a mixture of urea and biuret according to the present disclosure, hereinafter UB, with composition as in example 1 (41 % N, YARA International) on *in vitro* fermentation, nutrient digestibility, and methane production in a corn silage-based diet.

### *Approach and Research Procedures*

Two ruminally cannulated steers were used as ruminal fluid donors for the *in vitro* batch culture incubations. The steers were fed corn silage, cotton gin trash, and a premix of vitamins and minerals (70, 28, and 2 % on a dry matter basis, respectively) *ad libitum* at least 35 d before collecting ruminal fluid to perform the *in vitro* incubations. To adapt steers to the presence of NPN, each steer received the N equivalent to 100 g of urea per day (46 g of N daily/steer) but divided into two NPN sources. Thus, each steer was fed daily with 33 g of urea or 37 g of a urea/biuret mixture according to the present application. These amounts were mixed in with the diet daily. *In vitro* incubations were conducted on three separate days (replicates) using the same silage fed to the steers as the incubation substrate. The sources of NPN to be tested were analyzed for total N content and incubated on an isonitrogenous basis.

### *Treatments*

The incubation substrates (treatments) to test were control (CON, without NPN supplementation) and four increasing inclusions (from 1× to 4×) of the urea-biuret mixture according to the present application (UB). Treatments at 1× were designed to be iso-nitrogenous and equivalent to 1% of the inclusion in dry matter basis of urea (Yara International), in the diet, except for the CON treatment.

### *Batch culture incubations*

*In vitro* incubations were conducted on three separate days (replicates) using the same silage fed to the steers as the incubation substrate. A representative sample of rumen digesta was collected from different places in the rumen from the ruminally-cannulated steers and strained through 4 layers of cheesecloth, placed in pre-warmed thermos containers, and transported to the laboratory within 30 min of collection. In the laboratory, ruminal fluid from the two steers was maintained under constant $CO_2$ flux and was combined in equal proportions. Combined ruminal fluid was mixed with McDougall buffer in relation 1:4 (i.e., inoculum).

Two bottles of 120 mL per treatment containing 0.7 g and 50 mL of inoculum were incubated for 24 h at 39°C with gentle agitation (60 rpm). Further, two bottles without substrate were incubated as blank bottles. Gas pressure was recorded at 24 h, and a sample of gas was stored for further analysis. At the end of the 24 h of incubation, the final pH of fermentation fluid was recorded, and two 10-mL samples were collected and acidified by adding 20 $\mu$L of a 20% (vol/vol) of $H_2SO_4$ solution and were frozen at -20°C until further analysis.

Measurement of *in vitro* organic matter digestibility (IVOMD) was performed by incubating a separate set of duplicate 100-mL polypropylene tubes for 24 h at 39°C on each of the replicate days. These flasks contained 0.7 g of the substrate and were inoculated with 50 mL of a 4:1 McDougall's buffer:ruminal fluid mixture. After 24-h incubation with ruminal fluid, a pepsin-HCl solution was added, and tubes were incubated for another 48 h before filtering, drying at 105°C in a forced air oven for 24 h, and ashing at 550°C for 6 h to determine the undigested organic matter.

### *Sample analysis*

Concentrations of $NH_3$-N in the incubation fluid were measured after centrifuging at 10,000 x g for 15 min at 4°C (Avanti J-E, Beckman Coulter Inc., Palo Alto, CA) following the methodology described by Broderick and Kang (1980). Briefly, 1 mL of a phenol reagent was pipetted into 12 × 75 mm borosilicate disposable culture tubes (Fisherbrand; Thermo Fisher Scientific Inc., Waltham, MA). A 20-$\mu$L aliquot of the supernatant from the centrifuged sample was transferred to the phenol-containing culture tubes. After vortexing, 0.8 mL of a hypochlorite solution was added to the mixture and vortexed again. The culture tubes were covered with glass marbles and placed in a water bath at 95°C for 5 min. Absorbance was

read on 200 μL samples at $OD_{620}$ in flat-bottom 96-well plates (Corning Costar 3361, Thermo Fisher Scientific Inc., Waltham, MA) using a plate reader (Fisherbrand UV/VIS AccuSkan GO Spectrophotometer, Thermo Fisher Scientific Inc., Hampton, NH). All assays were conducted in duplicate determinations with subsequent analyses performed when CVs were above 5%.

Total gas production was measured using a manual transducer (Digital Test Gauge, Ashcroft Inc., Stratford, CT, USA), and a subsample was analyzed to measure $CH_4$ concentration by gas chromatography to calculate total $CH_4$ production during the course of the fermentation. To determine concentrations of $CH_4$, a gas chromatograph (Agilent 7820A GC, Agilent Technologies, Palo Alto, CA) using flame ionization and a capillary column (Plot Fused Silica 25 m × 0.32 mm, Coating Molsieve 5A, Varian CP7536; Varian Inc., Lake Forest, CA) was used. Temperatures of the injector, column, and detector were 80, 160, and 200°C, respectively, and $N_2$ was the carrier gas flowing at 3.3 mL/min. The split ratio for the injected $CH_4$ sample was 100:1.

Additionally, total volatile fatty acids (VFA) concentrations were measured by gas chromatography in the incubation fluid at the end of the fermentation to assess potential changes in the profile of VFA being produced, following the methodology described by Ruiz-Moreno et al. (2015). Briefly, samples were centrifuged at 10,000 × g for 15 min at 4°C. Two milliliters of the supernatant were mixed with 0.4 mL (5:1 ratio) of a metaphosphoric:crotonic acid (internal standard) solution, and samples were frozen overnight. Samples were thawed and centrifuged again at 10,000 × g for 15 min at 4°C. The supernatant was transferred into 12 mm × 75 mm borosilicate disposable culture tubes (Fisherbrand; Thermo Fisher Scientific Inc., Waltham, MA) and mixed with ethyl acetate to form a 2:1 ethyl acetate:supernatant mixture. Culture tubes were vigorously shaken and followed by a 5 min rest time to allow the separation of the ethyl acetate. A subsample of the ethyl acetate was transferred into small vials prior to analysis. Samples were analyzed with a gas chromatograph (Agilent 7820A GC, Agilent Technologies) using a flame ionization detector and a capillary column (CP-WAX 58 FFAP 25 m × 0.53 mm, Varian CP7767; Varian Inc.). The column temperature was maintained at 110°C, and the injector and detector temperatures were 200 and 220°C, respectively.

### Increasing dose of urea/biuret on in vitro fermentation and $CH_4$ emissions - results

The results are represented in Table 12.

Table 12. Effect of increasing the UB inclusion on fluid fermentation pH, the concentrations of volatile fatty acids and ammonia-N, in vitro organic matter digestibility, and gas and methane production in a corn silage-base diet.

| Variable[1] | UB inclusion | | | | |
|---|---|---|---|---|---|
| | 0x | 1x | 2x | 3x | 4x |
| pH | 6.50 | 6.49 | 6.54 | 6.59 | 6.63 |
| Total volatile fatty acids, mM | 88.72 | 105.78 | 101.83 | 99.92 | 96.03 |
| Acetate, mM | 55.57 | 65.41 | 52.45 | 61.03 | 59.15 |
| Propionate, mM | 18.19 | 22.12 | 21.14 | 21.09 | 19.68 |
| Butyrate, mM | 10.79 | 13.21 | 13.36 | 13.02 | 12.69 |
| Acetate, mol/100 mol | 62.64 | 61.76 | 61.32 | 61.00 | 61.56 |
| Propionate, mol/100 mol | 20.52 | 20.89 | 20.77 | 21.07 | 20.52 |
| Butyrate, mol/100 mol | 12.15 | 12.57 | 13.11 | 13.13 | 13.25 |
| Acetate:Propionate | 3.05 | 2.97 | 2.97 | 2.90 | 3.01 |
| Ammonia-N, mM | 3.25 | 6.96 | 9.96 | 13.88 | 15.39 |
| IVOMD, % | 54.40 | 54.96 | 55.13 | 58.77 | 57.48 |
| Gas production, mL/g OMd | 300.26 | 301.34 | 297.04 | 263.94 | 262.43 |
| Methane production, mM/g OMd | 33.73 | 32.74 | 32.43 | 28.84 | 23.02 |
| [1]IVOMD: In vitro organic matter digestibility; OMd: Organic matter degraded. | | | | | |

Increasing doses of UB linearly increased the concentration of $NH_3$-N, the rumen fluid pH, and the IVOMD. Also, the concentration of total VFA, acetate, propionate, and butyrate quadratically increased when increasing UB inclusion. Additionally, increasing the inclusion of UB linearly reduced the acetate:propionate ratrio and gas and $CH_4$ production (Table 12).

In the present experiment, the inclusion of UB increased the $NH_3$-N concentration resulting in greater IVOMD and concentration of total and individual VFA, indicative of a greater fermentation. Rumen microbes can utilize $NH_3$-N to synthezise microbial protein promoting greater fermentation. Additionally, the greater concentration of $NH_3$-N is in line with the linear increase in the rumen fluid pH.

Increasing the UB inclusion promoted other metabolic routes different than acetate formation, with a linear decrease in acetate proportion upon increasing the UB supplementation.

Furthermore, increasing the UB supplementation linearly decreased gas and $CH_4$ production but increased VFA and IVOMD, suggesting changes in rumen microbial communities, possibly Archaeas and fibrolytic bacteria, explaining the lower production of $CH_4$ and the lower proportion of acetate. Without wishing to be bound by theory, the greater concentration of $NH_3$-N during the incubation results in a greater fermentation rate, thereby increasing the concentration of VFA and IVOMD. However, the faster accumulation of $NH_3$-N may result in a toxic effect on some rumen microbial communities affecting the gas and $CH_4$ production.

**Claims**

1. Use of a composition comprising biuret, urea and N-containing by-products of the biuret production out of urea as a NPN-source in a ruminant feed or in a ruminant feed supplement composition for decreasing the enteric methane emissions of a ruminant animal, particularly when compared to a ruminant feed or a ruminant feed supplement composition comprising urea as a NPN-source, wherein the composition comprises between 30 and 60 wt.% of urea and between 30 and 60 wt.% biuret.

2. Use according to claim 1, for further and simultaneously decreasing the acetate-to-propionate ratio produced by rumen microbes in a ruminant animal, particularly when compared to a ruminant feed or a ruminant feed supplement composition comprising urea as a NPN-source.

3. Use according to claim 1 or 2, for further and simultaneously improving the efficiency of microbial protein synthesis (EMPS) by rumen microbes in a ruminant animal, particularly when compared to a ruminant feed or a ruminant feed supplement composition comprising urea as a NPN-source.

4. Use according to any one of claims 1 to 3, for further and simultaneously increasing the dry matter (DM) and organic matter (OM) digestibility, particularly when compared to a ruminant feed or a ruminant feed supplement composition comprising urea as a NPN-source.

5. Use according to any one of claims 1 to 4, for further and simultaneously increasing the dry matter intake (DMI) of a ruminant animal, particularly when compared to a ruminant feed or a ruminant feed supplement composition comprising urea as a NPN-source.

6. Use of a composition comprising biuret, urea and N-containing by-products of the biuret production out of urea as a NPN-source in a ruminant feed or in a ruminant feed supplement composition for improving rumen fermentation, wherein the acetate-to-propionate ratio produced by rumen microbes in a ruminant animal is decreased, particularly when compared to a ruminant feed or a ruminant feed supplement composition comprising urea as a NPN-source; and/or wherein the efficiency of microbial protein synthesis (EMPS) by rumen microbes in a ruminant animal is improved, particularly when compared to a ruminant feed or a ruminant feed supplement composition comprising urea as a NPN-source; more particularly for further and simultaneously increasing the dry matter (DM) and organic matter (OM) digestibility particularly when compared to a ruminant feed or a ruminant feed supplement composition comprising urea as a NPN-source; and/or increasing the dry matter intake (DMI) of a ruminant animal, particularly when compared to a ruminant feed or a ruminant feed supplement composition comprising urea as a NPN-source; wherein the composition comprises between 30 and 60 wt.% of urea and between 30 and 60 wt.% biuret, more in particular wherein the composition comprises between 35 and 55 wt.% of urea and between 35 and 50 wt.% of biuret, based on the total weight of the composition, and most in particular wherein the composition comprises between 37 and 52 wt.% of urea and between 39 and 46 wt.% of biuret, based on the total weight of the composition.

7. Use according to any one of claims 1 to 6, wherein the composition further comprises between 5 and 30 wt.% of N-containing by-products of the biuret production out of urea, based on the total weight of the composition, more in particular wherein the composition comprises between 2.5 and 8 wt.% of triuret, and/or between 0.1 and 5 wt.% of ammelide, and/or between 2.5 and 15 wt.% of cyanuric acid, and between 0.1 and 2.0 wt.% of moisture, based on the total weight of the composition.

8. Use according to claim 7, wherein the composition comprises between 3 and 6 wt.% of triuret, and/or between 0.5 and 3 wt.% of ammelide, and/or between 3 and 10 wt.% of cyanuric acid, and between 0.1 and 1.5 wt.% of moisture, based on the total weight of the composition.

9. A method for reducing the enteric methane emissions of a ruminant animal, comprising administering to the ruminant animal a NPN composition comprising biuret, urea and N-containing by-products of the biuret production, wherein the NPN composition comprises between 30 and 60 wt.% of urea and between 30 and 60 wt.% biuret, particularly wherein the NPN composition comprises between 35 and 55 wt.% of urea, between 35 and 50 wt.% of biuret, and between 5 and 30 wt.% of N-containing by-products of the biuret production out of urea, based on the total weight of the composition, particularly wherein the enteric methane emissions of the ruminant animal, when expressed as g methane/kg dry matter intake (DMI) or as g methane/kg organic matter intake, are reduced by at least 5%, more particularly by at least 10%, when the NPN composition is administered to a ruminant as NPN source, compared to a diet comprising urea as NPN source.

10. The method according to claim 9, wherein said method is further simultaneously a method for:

- decreasing the acetate-to-propionate ratio produced by rumen microbes in a ruminant animal, particularly wherein the acetate-to-propionate ratio produced by rumen microbes in a ruminant, particularly determined via an *in vitro* continuous flow fermentation with rumen fluid to simulate rumen fermentation, is decreased by at least 5%, in particular by at least 10%, when the NPN composition is administered to a ruminant as NPN source compared to a diet comprising urea as NPN source,
- improving the efficiency of microbial protein synthesis (EMPS) by rumen microbes in a ruminant animal, wherein the EMPS, particularly determined via an *in vitro* continuous or batch rumen fluid incubation to simulate rumen fermentation, was increased by at least 5%, in particular by at least 10%, when the NPN composition is administered to a ruminant as NPN source compared to a diet comprising urea as NPN source;
- increasing the dry matter (DM) and organic matter (OM) digestibility; and/or
- increasing the dry matter intake (DMI) of a ruminant animal, particularly wherein the DMI, expressed in kg/day is increased by at least 5% when the NPN composition is administered to a ruminant as NPN source compared to a diet comprising urea as NPN source.

11. The method according to claim 9 or 10, wherein the NPN composition comprises between 35 and 55 wt.% of urea and between 35 and 50 wt.% of biuret, based on the total weight of the composition, more in particular comprises between 37 and 52 wt.% of urea and between 39 and 46 wt.% of biuret, based on the total weight of the composition.

12. The method according to any one of claims 9 to 11, wherein the NPN composition further comprises between 5 and 30 wt.% of N-containing by-products of the biuret production out of urea, based on the total weight of the composition, more in particular wherein the NPN composition comprises between 2.5 and 8 wt.% of triuret, and/or between 0.1 and 5 wt.% of ammelide, and/or between 2.5 and 15 wt.% of cyanuric acid, and between 0.1 and 2.0 wt.% of moisture, based on the total weight of the composition, even more in particular, wherein the composition comprises between 3 and 6 wt.% of triuret, and/or between 0.5 and 3 wt.% of ammelide, and/or between 3 and 10 wt.% of cyanuric acid, and between 0.1 and 1.5 wt.% of moisture, based on the total weight of the composition.

13. The method according to any one of claims 9 to 12, comprising the steps of preparing or providing a ruminant feed supplement composition comprising the NPN composition, or preparing or providing a ruminant feed composition comprising the NPN composition, and administering the ruminant feed supplement or the ruminant feed composition to the ruminant animal.

14. The method according to claim 13, wherein the ruminant feed composition further comprises one or more nutritional ingredients, wherein the one or more nutritional ingredients form a nutritionally balanced ration or a basal ration for a ruminant.

15. The use according to any one of claims 1 to 8, or the method according to claim 13, wherein the NPN composition is present in the ruminant feed composition in a concentration between 0.5 and 4 wt.%, more in particular between 0.5 and 3.5 wt.% or between 0.5 and 3 wt.%, even more in particular between 1 and 2.5 wt.%, with wt% based on the total dry matter basis of the ruminant feed composition.

**Patentansprüche**

1. Verwendung einer Zusammensetzung, umfassend Biuret, Harnstoff und N-haltige Nebenprodukte der Biuretproduktion aus Harnstoff, als NPN-Quelle in einem Futtermittel für Wiederkäuer oder in einer Futterergänzungszusammensetzung für Wiederkäuer zur Verringerung der enterischen Methanemissionen eines Wiederkäuers, besonders im Vergleich zu einem Futtermittel für Wiederkäuer bzw. einer Futterergänzungszusammensetzung für Wiederkäuer, das bzw. die Harnstoff als NPN-Quelle umfasst, wobei die Zusammensetzung zwischen 30 und 60 Gew.-% Harnstoff und zwischen 30 und 60 Gew.-% Biuret umfasst.

2. Verwendung nach Anspruch 1 zur weiteren und gleichzeitigen Verringerung des durch Pansenmikroben erzeugten Verhältnisses von Acetat zu Propionat in einem Wiederkäuer, besonders im Vergleich zu einem Futtermittel für Wiederkäuer bzw. einer Futterergänzungszusammensetzung für Wiederkäuer, das bzw. die Harnstoff als NPN-Quelle umfasst.

3. Verwendung nach Anspruch 1 oder 2 zur weiteren und gleichzeitigen Verbesserung der Effizienz von mikrobieller Proteinsynthese (EMPS) durch Pansenmikroben in einem Wiederkäuer, besonders im Vergleich zu einem Futtermittel für Wiederkäuer bzw. einer Futterergänzungszusammensetzung für Wiederkäuer, das bzw. die Harnstoff als NPN-Quelle umfasst.

4. Verwendung nach einem der Ansprüche 1 bis 3 zur weiteren und gleichzeitigen Erhöhung der Verdaulichkeit von Trockensubstanz (Dry Matter, DM) und organischer Substanz (Organic Matter, OM), besonders im Vergleich zu einem Futtermittel für Wiederkäuer bzw. einer Futterergänzungszusammensetzung für Wiederkäuer, das bzw. die Harnstoff als NPN-Quelle umfasst.

5. Verwendung nach einem der Ansprüche 1 bis 4 zur weiteren und gleichzeitigen Erhöhung der Trockensubstanzaufnahme (Dry Matter Intake, DMI) eines Wiederkäuers, besonders im Vergleich zu einem Futtermittel für Wiederkäuer bzw. einer Futterergänzungszusammensetzung für Wiederkäuer, das bzw. die Harnstoff als NPN-Quelle umfasst.

6. Verwendung einer Zusammensetzung, umfassend Biuret, Harnstoff und N-haltige Nebenprodukte der Biuretproduktion aus Harnstoff, als NPN-Quelle in einem Futtermittel für Wiederkäuer oder in einer Futterergänzungszusammensetzung für Wiederkäuer zur Verbesserung der Pansenfermentation, wobei das durch Pansenmikroben erzeugte Verhältnis von Acetat zu Propionat in einem Wiederkäuer verringert wird, besonders im Vergleich zu einem Futtermittel für Wiederkäuer bzw. einer Futterergänzungszusammensetzung für Wiederkäuer, das bzw. die Harnstoff als NPN-Quelle umfasst; und/oder wobei die Effizienz der mikrobiellen Proteinsynthese (EMPS) durch Pansenmikroben in einem Wiederkäuer verbessert wird, besonders im Vergleich zu einem Futtermittel für Wiederkäuer bzw. einer Futterergänzungszusammensetzung für Wiederkäuer, das bzw. die Harnstoff als NPN-Quelle umfasst; ganz besonders zur weiteren und gleichzeitigen Erhöhung der Verdaulichkeit von Trockensubstanz (DM) und organischer Substanz (OM), besonders im Vergleich zu einem Futtermittel für Wiederkäuer bzw. einer Futterergänzungszusammensetzung für Wiederkäuer, das bzw. die Harnstoff als NPN-Quelle umfasst; und/oder zur weiteren und gleichzeitigen Erhöhung der Trockensubstanzaufnahme (DMI) eines Wiederkäuers, besonders im Vergleich zu einem Futtermittel für Wiederkäuer bzw. einer Futterergänzungszusammensetzung für Wiederkäuer, das bzw. die Harnstoff als NPN-Quelle umfasst;
wobei die Zusammensetzung zwischen 30 und 60 Gew.-% Harnstoff und zwischen 30 und 60 Gew.-% Biuret umfasst, weiter insbesondere wobei die Zusammensetzung zwischen 35 und 55 Gew.-% Harnstoff und zwischen 35 und 50 Gew.-% Biuret, jeweils bezogen auf das Gesamtgewicht der Zusammensetzung, umfasst und vor allem wobei die Zusammensetzung zwischen 37 und 52 Gew.-% Harnstoff und zwischen 39 und 46 Gew.-% Biuret, jeweils bezogen auf das Gesamtgewicht der Zusammensetzung, umfasst.

7. Verwendung nach einem der Ansprüche 1 bis 6, wobei die Zusammensetzung ferner zwischen 5 und 30 Gew.-% N-haltige Nebenprodukte der Biuretproduktion aus Harnstoff, jeweils bezogen auf das Gesamtgewicht der Zusammensetzung, umfasst, weiter insbesondere wobei die Zusammensetzung zwischen 2,5 und 8 Gew.-% Triuret und/oder zwischen 0,1 und 5 Gew.-% Ammelid und/oder zwischen 2,5 und 15 Gew.-% Cyanursäure sowie zwischen 0,1 und 2,0 Gew.-% Feuchtigkeit, jeweils bezogen auf das Gesamtgewicht der Zusammensetzung, umfasst.

8. Verwendung nach Anspruch 7, wobei die Zusammensetzung zwischen 3 und 6 Gew.-% Triuret und/oder zwischen 0,5 und 3 Gew.-% Ammelid und/oder zwischen 3 und 10 Gew.-% Cyanursäure sowie zwischen 0,1 und 1,5 Gew.-% Feuchtigkeit, jeweils bezogen auf das Gesamtgewicht der Zusammensetzung, umfasst.

9. Verfahren zur Reduzierung der enterischen Methanemissionen eines Wiederkäuers, umfassend Verabreichen einer NPN-Zusammensetzung, die Biuret, Harnstoff und N-haltige Nebenprodukte der Biuretproduktion umfasst, an den Wiederkäuer, wobei die NPN-Zusammensetzung zwischen 30 und 60 Gew.-% Harnstoff und zwischen 30 und 60 Gew.-% Biuret umfasst, besonders wobei die NPN-Zusammensetzung zwischen 35 und 55 Gew.-% Harnstoff, zwischen 35 und 50 Gew.-% Biuret und zwischen 5 und 30 Gew.-% N-haltige Nebenprodukte der Biuretproduktion aus Harnstoff, jeweils bezogen auf das Gesamtgewicht der Zusammensetzung, umfasst, besonders wobei die enterischen Methanemissionen des Wiederkäuers, ausgedrückt als g Methan pro kg Trockensubstanzaufnahme (DMI) oder als g Methan pro kg Aufnahme organischer Substanz, um mindestens 5 %, ganz besonders um mindestens 10 % reduziert werden, wenn einem Wiederkäuer die NPN-Zusammensetzung als NPN-Quelle verabreicht wird, verglichen mit einer Harnstoff als NPN-Quelle umfassenden Fütterung.

10. Verfahren nach Anspruch 9, wobei es sich bei dem Verfahren weiter gleichzeitig um ein Verfahren für Folgendes handelt:

- Verringern des durch Pansenmikroben in einem Wiederkäuer erzeugten Verhältnisses von Acetat zu Propionat, besonders wobei das durch Pansenmikroben in einem Wiederkäuer erzeugte Verhältnis von Acetat zu Propionat, besonders über eine In-vitro-Fermentation mit kontinuierlichem Fluss mit Pansenflüssigkeit zur Simulierung von Pansenfermentation bestimmt, um mindestens 5 %, insbesondere um mindestens 10 % verringert wird, wenn einem Wiederkäuer die NPN-Zusammensetzung als NPN-Quelle verabreicht wird, verglichen mit einer Harnstoff als NPN-Quelle umfassenden Fütterung,
- Verbessern der Effizienz von mikrobieller Proteinsynthese (EMPS) durch Pansenmikroben in einem Wiederkäuer, wobei die EMPS, besonders über eine kontinuierliche oder chargenweise In-vitro-Pansenflüssigkeitsinkubation zur Simulierung von Pansenfermentation bestimmt, um mindestens 5 %, insbesondere um mindestens 10 % erhöht wurde, wenn einem Wiederkäuer die NPN-Zusammensetzung als NPN-Quelle verabreicht wird, verglichen mit einer Harnstoff als NPN-Quelle umfassenden Fütterung;
- Erhöhen der Verdaulichkeit von Trockensubstanz (DM) und organischer Substanz (OM); und/oder
- Erhöhen der Trockensubstanzaufnahme (DMI) eines Wiederkäuers, besonders wobei die DMI, ausgedrückt in kg/Tag, um mindestens 5 % erhöht wird, wenn einem Wiederkäuer die NPN-Zusammensetzung als NPN-Quelle verabreicht wird, verglichen mit einer Harnstoff als NPN-Quelle umfassenden Fütterung.

11. Verfahren nach Anspruch 9 oder 10, wobei die NPN-Zusammensetzung zwischen 35 und 55 Gew.-% Harnstoff und zwischen 35 und 50 Gew.-% Biuret, jeweils bezogen auf das Gesamtgewicht der Zusammensetzung, weiter insbesondere zwischen 37 und 52 Gew.-% Harnstoff und zwischen 39 und 46 Gew.-% Biuret, jeweils bezogen auf das Gesamtgewicht der Zusammensetzung, umfasst.

12. Verfahren nach einem der Ansprüche 9 bis 11, wobei die NPN-Zusammensetzung ferner zwischen 5 und 30 Gew.-% N-haltige Nebenprodukte der Biuretproduktion aus Harnstoff, bezogen auf das Gesamtgewicht der Zusammensetzung, umfasst, weiter insbesondere wobei die NPN-Zusammensetzung zwischen 2,5 und 8 Gew.-% Triuret und/oder zwischen 0,1 und 5 Gew.-% Ammelid und/oder zwischen 2,5 und 15 Gew.-% Cyanursäure sowie zwischen 0,1 und 2,0 Gew.-% Feuchtigkeit, jeweils bezogen auf das Gesamtgewicht der Zusammensetzung, umfasst, noch weiter insbesondere wobei die Zusammensetzung zwischen 3 und 6 Gew.-% Triuret und/oder zwischen 0,5 und 3 Gew.-% Ammelid und/oder zwischen 3 und 10 Gew.-% Cyanursäure sowie zwischen 0,1 und 1,5 Gew.-% Feuchtigkeit, jeweils bezogen auf das Gesamtgewicht der Zusammensetzung, umfasst.

13. Verfahren nach einem der Ansprüche 9 bis 12, umfassend die Schritte Herstellen oder Bereitstellen einer die NPN-Zusammensetzung umfassenden Futterergänzungszusammensetzung für Wiederkäuer oder Herstellen oder Bereitstellen einer die NPN-Zusammensetzung umfassenden Futtermittelzusammensetzung für Wiederkäuer und Verabreichen der Futterergänzungs- bzw. Futtermittelzusammensetzung für Wiederkäuer an den Wiederkäuer.

14. Verfahren nach Anspruch 13, wobei die Futtermittelzusammensetzung für Wiederkäuer ferner einen oder mehrere Nährstoffe umfasst, wobei die ein oder mehreren Nährstoffe eine nährstoffmäßig ausgewogene Ration oder eine Grundration für einen Wiederkäuer bilden.

15. Verwendung nach einem der Ansprüche 1 bis 8 oder Verfahren nach Anspruch 13, wobei die NPN-Zusammensetzung in der Futtermittelzusammensetzung für Wiederkäuer in einer Konzentration zwischen 0,5 und 4 Gew.-%, weiter insbesondere zwischen 0,5 und 3,5 Gew.-% oder zwischen 0,5 und 3 Gew.-%, noch weiter insbesondere zwischen 1 und 2,5 Gew.-% vorliegt, wobei sich Gew.-% jeweils auf die Gesamttrockensubstanzbasis der Futtermittelzusammensetzung für Wiederkäuer beziehen.

**Revendications**

1. Utilisation d'une composition comprenant du biuret, de l'urée et des sous-produits contenant de l'azote de la production de biuret à partir d'urée en tant que source de NPN dans un aliment pour ruminants ou dans une composition de complément alimentaire pour ruminants pour diminuer les émissions de méthane entérique d'un animal ruminant, en particulier par rapport à un aliment pour ruminants ou une composition de complément alimentaire pour ruminants comprenant de l'urée en tant que source de NPN, dans laquelle la composition comprend entre 30 et 60 % en poids d'urée et entre 30 et 60 % en poids de biuret.

2. Utilisation selon la revendication 1, pour diminuer en outre et simultanément le rapport acétate-propionate produit par les microbes du rumen chez un animal ruminant, en particulier par rapport à un aliment pour ruminants ou à une composition de complément alimentaire pour ruminants comprenant de l'urée comme source de NPN.

3. Utilisation selon la revendication 1 ou 2, pour améliorer en outre et simultanément l'efficacité de la synthèse de protéines microbiennes (EMPS) par des microbes du rumen chez un animal ruminant, en particulier par rapport à un aliment pour ruminants ou à une composition de complément alimentaire pour ruminants comprenant de l'urée comme source de NPN.

4. Utilisation selon l'une quelconque des revendications 1 à 3, pour augmenter en outre et simultanément la digestibilité de la matière sèche (DM) et de la matière organique (OM), en particulier par rapport à un aliment pour ruminants ou à une composition de complément alimentaire pour ruminants comprenant de l'urée comme source de NPN.

5. Utilisation selon l'une quelconque des revendications 1 à 4, pour augmenter en outre et simultanément l'apport en matière sèche (DMI) d'un animal ruminant, en particulier par rapport à un aliment pour ruminants ou à une composition de complément alimentaire pour ruminants comprenant de l'urée comme source de NPN.

6. Utilisation d'une composition comprenant du biuret, de l'urée et des sous-produits contenant de l'azote de la production de biuret à partir d'urée comme source de NPN dans un aliment pour ruminants ou dans une composition de complément alimentaire pour ruminants pour améliorer la fermentation du rumen, dans laquelle le rapport acétate-propionate produit par les microbes du rumen chez un animal ruminant est diminué, en particulier par rapport à un aliment pour ruminants ou à une composition de complément alimentaire pour ruminants comprenant de l'urée comme source de NPN ; et/ou dans laquelle l'efficacité de la synthèse de protéines microbiennes (EMPS) par les microbes du rumen chez un animal ruminant est améliorée, en particulier par rapport à un aliment pour ruminants ou à une composition de complément alimentaire pour ruminants comprenant de l'urée comme source de NPN ; plus particulièrement pour augmenter en outre et simultanément la digestibilité de matière sèche (DM) et de matière organique (OM), en particulier par rapport à un aliment pour ruminants ou à une composition de complément alimentaire pour ruminants comprenant de l'urée comme source de NPN ; et/ou augmenter l'apport en matière sèche (DMI) d'un animal ruminant, en particulier par rapport à un aliment pour ruminants ou à une composition de complément alimentaire pour ruminants comprenant de l'urée comme source de NPN ; dans laquelle la composition comprend entre 30 et 60 % en poids d'urée et entre 30 et 60 % en poids de biuret, plus particulièrement dans laquelle la composition comprend entre 35 et 55 % en poids d'urée et entre 35 et 50 % en poids de biuret, par rapport au poids total de la composition, et tout particulièrement dans laquelle la composition comprend entre 37 et 52 % en poids d'urée et entre 39 et 46 % en poids de biuret, par rapport au poids total de la composition.

7. Utilisation selon l'une quelconque des revendications 1 à 6, dans laquelle la composition comprend en outre entre 5 et 30 % en poids de sous-produits contenant de l'azote de la production de biuret à partir d'urée, par rapport au poids total de la composition, plus particulièrement dans laquelle la composition comprend entre 2,5 et 8 % en poids de triuret, et/ou entre 0,1 et 5 % en poids d'ammélide, et/ou entre 2,5 et 15 % en poids d'acide cyanurique, et entre 0,1 et 2,0 % en poids d'humidité, par rapport au poids total de la composition.

8. Utilisation selon la revendication 7, dans laquelle la composition comprend entre 3 et 6 % en poids de triuret, et/ou entre 0,5 et 3 % en poids d'ammélide, et/ou entre 3 et 10 % en poids d'acide cyanurique, et entre 0,1 et 1,5 % en poids d'humidité, par rapport au poids total de la composition.

9. Procédé pour réduire les émissions de méthane entérique d'un animal ruminant, comprenant l'administration à l'animal ruminant d'une composition NPN comprenant du biuret, de l'urée et des sous-produits contenant de l'azote de la production de biuret, dans lequel la composition NPN comprend entre 30 et 60 % en poids d'urée et entre 30 et 60 % en poids de biuret, en particulier dans lequel la composition NPN comprend entre 35 et 55 % en poids d'urée, entre 35 et 50 % en poids de biuret, et entre 5 et 30 % en poids de sous-produits contenant de l'azote de la production de biuret à partir d'urée, par rapport au poids total de la composition, en particulier dans lequel les émissions de méthane entérique de l'animal ruminant, exprimées en g de méthane/kg d'apport en matière sèche (DMI) ou en g de méthane/kg d'apport en matière organique, sont réduites d'au moins 5 %, plus particulièrement d'au moins 10 %, lorsque la composition NPN est administrée à un animal ruminant en tant que source de NPN, par rapport à un régime comprenant de l'urée comme source de NPN.

**10.** Procédé selon la revendication 9, dans lequel ledit procédé est en outre simultanément un procédé pour :

- diminuer le rapport acétate-propionate produit par des microbes du rumen chez un ruminant, en particulier dans lequel le rapport acétate sur propionate produit par les microbes du rumen chez un animal ruminant, en particulier déterminé par une fermentation en flux continu *in vitro* avec du liquide du rumen pour simuler la fermentation du rumen, est diminué d'au moins 5 %, en particulier d'au moins 10 %, lorsque la composition NPN est administrée à un ruminant en tant que source de NPN par rapport à un régime comprenant de l'urée en tant que source de NPN,
- améliorer l'efficacité de la synthèse de protéines microbiennes (EMPS) par les microbes du rumen chez un animal ruminant, dans lequel l'EMPS, déterminée en particulier par incubation continue ou discontinue *in vitro* de liquide du rumen pour simuler la fermentation du rumen, a été augmentée d'au moins 5%, en particulier d'au moins 10 %, lorsque la composition NPN est administrée à un ruminant en tant que source de NPN par rapport à un régime comprenant de l'urée en tant que source de NPN ;
- augmenter la digestibilité de la matière sèche (DM) et de la matière organique (OM) ; et/ou
- augmenter l'apport en matière sèche (DMI) d'un animal ruminant, en particulier dans lequel la DMI, exprimée en kg/jour, est augmentée d'au moins 5 % lorsque la composition NPN est administrée à un ruminant en tant que source de NPN par rapport à un régime alimentaire comprenant de l'urée en tant que source de NPN.

**11.** Procédé selon la revendication 9 ou 10, dans lequel la composition NPN comprend entre 35 et 55 % en poids d'urée et entre 35 et 50 % en poids de biuret, par rapport au poids total de la composition, plus particulièrement entre 37 et 52 % en poids d'urée et entre 39 et 46 % en poids de biuret, par rapport au poids total de la composition.

**12.** Procédé selon l'une quelconque des revendications 9 à 11, dans lequel la composition NPN comprend en outre entre 5 et 30 % en poids de sous-produits contenant de l'azote de la production de biuret à partir d'urée, par rapport au poids total de la composition, plus particulièrement dans lequel la composition NPN comprend entre 2,5 et 8 % en poids de triuret, et/ou entre 0,1 et 5 % en poids d'ammélide, et/ou entre 2,5 et 15 % en poids d'acide cyanurique, et entre 0,1 et 2,0 % en poids d'humidité, par rapport au poids total de la composition, encore plus particulièrement, dans lequel la composition comprend entre 3 et 6 % en poids de triuret, et/ou entre 0,5 et 3 % en poids d'ammélide, et/ou entre 3 et 10 % en poids d'acide cyanurique, et entre 0,1 et 1,5 % en poids d'humidité, par rapport au poids total de la composition.

**13.** Procédé selon l'une quelconque des revendications 9 à 12, comprenant les étapes de préparation ou de fourniture d'une composition de complément alimentaire pour ruminants comprenant la composition NPN, ou de préparation ou de fourniture d'une composition alimentaire pour ruminants comprenant la composition NPN, et d'administration du complément alimentaire pour ruminants ou de la composition alimentaire pour ruminants à l'animal ruminant.

**14.** Procédé selon la revendication 13, dans lequel la composition alimentaire pour ruminants comprend en outre un ou plusieurs ingrédients nutritionnels, dans lequel le ou les ingrédients nutritionnels forment une ration équilibrée sur le plan nutritionnel ou une ration de base pour un ruminant.

**15.** Utilisation selon l'une quelconque des revendications 1 à 8, ou procédé selon la revendication 13, dans laquelle/lequel la composition NPN est présente dans la composition alimentaire pour ruminants en une concentration comprise entre 0,5 et 4 % en poids, plus particulièrement entre 0,5 et 3,5 % en poids ou entre 0,5 et 3 % en poids, encore plus particulièrement entre 1 et 2,5 % en poids, les % en poids étant basés sur la matière sèche totale de la composition alimentaire pour ruminants.

FIG. 1

# FIG. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Effects of urea plus nitrate pretreated rice straw and corn oil supplementation on fiber digestibility, nitrogen balance, rumen fermentation, microbiota and methane emissions in goats. **XIUMIN ZHANG et al.** Journal of Animal Science and Biotechnology. Biomed, Central Ltd, 23 January 2019, vol. 10, 1-10

- **JOUANY, J. P.** ; **J. SENAUD.** Influence des ciliés du rumen sur la digestion de différents glucides chez le mouton. I.-Utilisation des glucides pariétaux (cellulose et hémicelluloses) et de l'amidon. *Reproduction Nutrition Développement*, 1982, vol. 22 (5), 735-752
- **VAN SOEST et al.** Ankom 200 Fiber Analyzer. Ankom Technology Corp., 1991
- Avanti J-E. Beckman Coulter Inc.